(19) Europäisches Patentamt
European Patent Office
Office européen des brevets

(11) Publication number: 0 354 693 A1

(12) # EUROPEAN PATENT APPLICATION

(21) Application number: 89307724.8

(22) Date of filing: 28.07.89

(51) Int. Cl.⁴: C07D 491/052 , A61K 31/415 , //(C07D491/052,335:00,231:00)

Claims for the following Contracting States: ES + GR.

(30) Priority: 09.08.88 GB 8818894

(43) Date of publication of application: 14.02.90 Bulletin 90/07

(84) Designated Contracting States: AT BE CH DE ES FR GB GR IT LI LU NL SE

(71) Applicant: THE BOOTS COMPANY PLC
1 Thane Road West
Nottingham NG2 3AA(GB)

(72) Inventor: Bowen, John Gareth
Cherrytrees Barkestone Lane
Plungar Notts(GB)
Inventor: Hockley, Michael Henry
18 Amilda Avenue
Ilkeston Derbyshire(GB)
Inventor: Housley, John Rosindale
146 Wilsthorpe Road
Breaston Derbyshire(GB)
Inventor: Hunneyball, Ian Michael
8 Valley Road
Radcliffe-on-Trent Notts(GB)
Inventor: Titman, Roger Bernard
29 Linwood Crescent
Ravenshead Nottingham(GB)
Inventor: Webber, Dabid George
8 Moss Close
East Bridgford Nottingham(GB)

(74) Representative: Thacker, Michael Anthony et al
THE BOOTS COMPANY PLC Patents Section
R4 Pennyfoot Street
Nottingham NG2 3AA(GB)

(54) **Therapeutic agents.**

(57) This invention relates to compounds of formula I

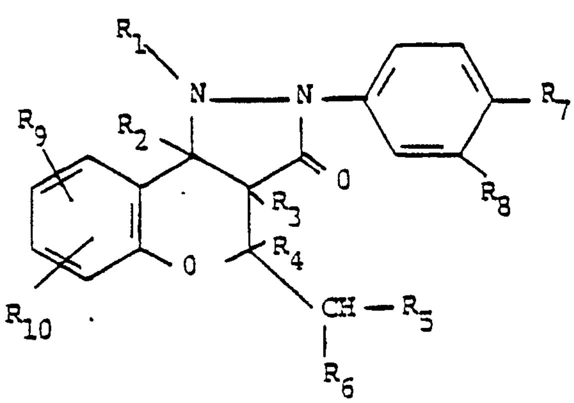

I

wherein $R_1$ represents hydrogen or together with $R_2$ represents a bond; $R_2$ together with either one of $R_1$ and $R_3$ represents a bond; $R_3$ together with either one of $R_2$ and $R_4$ represents a bond; $R_4$ represents hydrogen or together with $R_3$ represents a bond; $R_5$ represents hydrogen or methyl; $R_6$ represents hydrogen, halo, a $C_{2-6}$ alkanoyl group, a $C_{2-6}$ alkoxycarbonyl group, a $C_{1-6}$ alkylthio group, a $C_{1-6}$

EP 0 354 693 A1

alkylsulphinyl group, a $C_{1-6}$ alkylsulphonyl group, carbamoyl, carboxy, or $R_5$ and $R_6$ together with the carbon atom to which they are attached represent a cyclopropyl group; $R_7$ represents hydrogen, halo, trifluoromethyl, methoxy, a $C_{1-6}$ alkyl group, a $C_{1-6}$ alkylthio group or a $C_{1-6}$ alkylsulphinyl group; $R_8$ represents hydrogen, halo or trifluoromethyl; $R_9$ and $R_{10}$, which may be the same or different, each represent halo; or $R_9$ represents hydrogen and $R_{10}$ represents hydrogen, halo, trifluoromethyl, hydroxy, nitro, a $C_{2-6}$ alkanoyloxy group, a $C_{1-6}$ alkyl group or a $C_{1-6}$ alkoxy group, which have immunomodulatory activity. Compositions containing these compounds and processes to make them are also disclosed.

# THERAPEUTIC AGENTS

The present invention relates to novel therapeutic agents, and in particular to [1]benzopyrano[4,3-c]-pyrazoles, to processes for their preparation, to pharmaceutical compositions containing them and to their therapeutic activity as immunomodulatory agents.

It is disclosed in US Patent 4268516 that certain compounds are capable of suppressing the immune response in mammals. These compounds form a small group of 1,2,3,4-tetrahydro[1]benzothiopyrano[4,3-c]pyrazol-3-ones and 2,3-dihydro[1]benzothiopyrano[4,3-c]pyrazol-3-ones of respective structures A and B

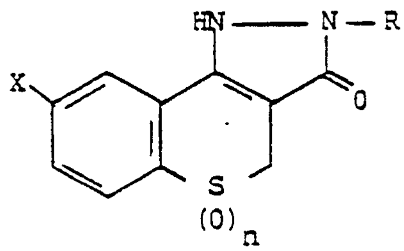

A

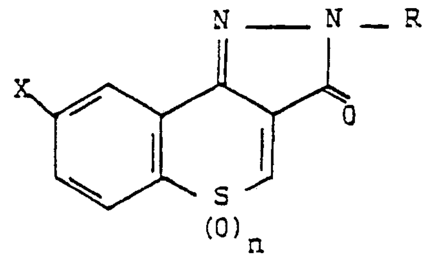

B

wherein n is an integer of value from 0 to 2; R is phenyl; phenyl monosubstituted with chloro, bromo, fluoro, methyl, methoxy, nitro or trifluoromethyl; or phenyl disubstituted with chloro; and X is hydrogen or chloro.

The present invention relates to compounds of formula I

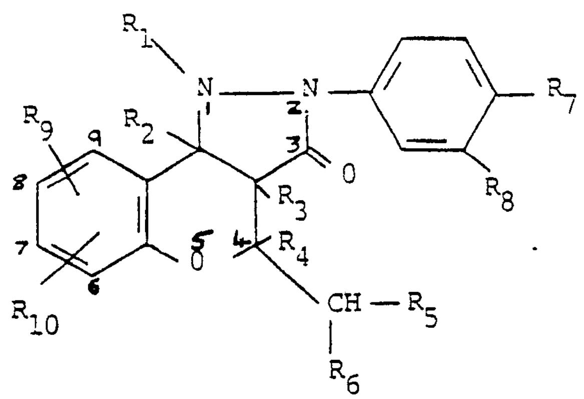

I

wherein $R_1$ represents hydrogen or together with $R_2$ represents a bond; $R_2$ together with either one of $R_1$ and $R_3$ represents a bond; $R_3$ together with either one or $R_2$ and $R_4$ represents a bond; $R_4$ represents hydrogen or together with $R_3$ represents a bond; $R_5$ represents hydrogen or methyl; $R_6$ represents hydrogen, halo, a $C_{2-6}$ alkanoyl group, a $C_{2-6}$ alkoxycarbonyl group, a $C_{1-6}$ alkylthio group, a $C_{1-6}$ alkylsulphinyl group, a $C_{1-6}$ alkylsulphonyl group, carbamoyl, carboxy or $R_5$ and $R_6$ together with the carbon atom to which they are attached represent a cyclopropyl group; $R_7$ represents hydrogen, halo, trifluoromethyl, methoxy, a $C_{1-6}$ alkyl group, a $C_{1-6}$ alkylthio group or a $C_{1-6}$ alkylsulphinyl group; $R_8$ represents hydrogen, halo or trifluoromethyl; $R_9$ and $R_{10}$, which may be the same or different, each represent halo; or $R_9$ represents hydrogen and $R_{10}$ represents hydrogen, halo, trifluoromethyl, hydroxy, nitro, a $C_{2-6}$ alkanoyloxy group, a $C_{1-6}$ alkyl group or a $C_{1-6}$ alkoxy group, which have been found to have immunomodulatory activity.

In Tetrahedron Letters, 1987, Vol. 28, No. 43 pp5165-5168 there is described a compound of formula I in which $R_1$ and $R_2$ represent a bond, $R_3$ and $R_4$ represent a bond, and $R_5$, $R_6$, $R_7$, $R_8$, $R_9$ and $R_{10}$ each represent hydrogen. However this document discloses no pharmaceutical activity in respect of this compound.

The present invention provides novel compounds of formula I wherein $R_1$ represents hydrogen or together with $R_2$ represents a bond; $R_2$ together with either one of $R_1$ and $R_3$ represents a bond; $R_3$ together with either one of $R_2$ and $R_4$ represents a bond; $R_4$ represents hydrogen or together with $R_3$ represents a bond; $R_5$ represents hydrogen or methyl; $R_6$ represents hydrogen, halo, a $C_{2-6}$ alkanoyl group, a $C_{2-6}$ alkoxycarbonyl group, a $C_{1-6}$ alkylthio group, a $C_{1-6}$ alkylsulphinyl group, a $C_{1-6}$

alkylsulphonyl group, carbamoyl, carboxy or $R_5$ and $R_6$ together with the carbon atom to which they are attached represent a cyclopropyl group; $R_7$ represents hydrogen, halo, trifluoromethyl, methoxy, a $C_{1-6}$ alkyl group, a $C_{1-5}$ alkylthio group or a $C_{1-6}$ alkylsulphinyl group; $R_8$ represents hydrogen, halo or trifluoromethyl; $R_9$ and $R_{10}$, which may be the same or different, each represent halo; or $R_9$ represents hydrogen and $R_{10}$ represents hydrogen, halo, trifluoromethyl, hydroxy, nitro, a $C_{2-6}$ alkanoyloxy group, a $C_{1-6}$ alkyl group or a $C_{1-6}$ alkoxy group provided that when $R_1$ and $R_2$ represent a bond, $R_3$ and $R_4$ represent a bond, and $R_6$, $R_7$, $R_8$, $R_9$ and $R_{10}$ each represent hydrogen then $R_5$ is not hydrogen.

It will be understood that a group containing a chain of 3 or more carbon atoms may be straight or branched, for example propyl includes n-propyl and isopropyl, and butyl includes n-butyl, sec-butyl, isobutyl and tert-butyl. The term "halo" covers fluoro, chloro or bromo.

A preferred group of compounds of formula I, in which $R_1$ and $R_2$ represent a bond and $R_3$ and $R_4$ represent a bond, is represented by formula II

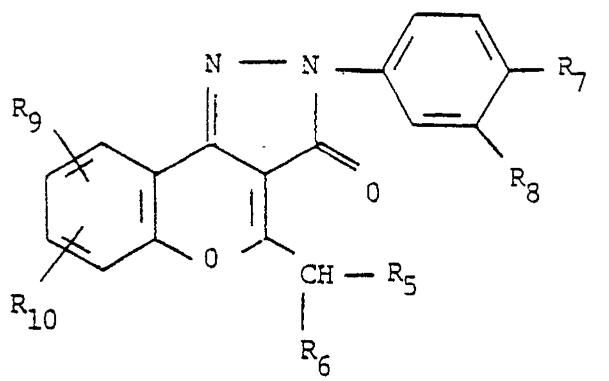

II

wherein $R_5$, $R_6$, $R_7$, $R_8$, $R_9$ and $R_{10}$ are as hereinbefore defined.

A further preferred group of compounds of formula I in which $R_1$ represents hydrogen, $R_2$ and $R_3$ represent a bond and $R_4$ represents hydrogen, is represented by formula III or tautomers thereof

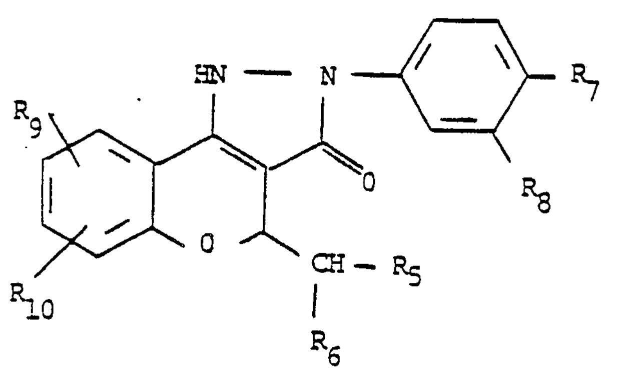

III

wherein $R_5$, $R_6$, $R_7$, $R_8$, $R_9$ and $R_{10}$ are as hereinbefore defined.

In compounds of formula I, when $R_5$ represents methyl, preferably $R_6$ represents a substituent other than hydrogen, for example a $C_{1-6}$ alkoxycarbonyl group. In preferred compounds of formula I, $R_5$ represents hydrogen or together with $R_6$ and the carbon atom to which they are attached represents a cyclopropyl group. More preferably $R_5$ represents hydrogen.

Preferably $R_6$ represents hydrogen, halo, a $C_{2-4}$ alkanoyl group (for example acetyl, ethanoyl or propanoyl) a $C_{2-6}$ alkoxycarbonyl group (for example methoxycarbonyl, ethoxycarbonyl, propoxycarbonyl or butoxycarbonyl), a $C_{1-4}$ alkylthio group (for example methylthio, ethylthio or propylthio), a $C_{1-4}$ alkylsulphinyl group (for example methylsulphinyl, ethylsulphinyl or propylsulphinyl, a $C_{1-4}$ alkylsulphonyl group (for example methylsulphonyl, ethylsulphonyl or propylsulphonyl), carbamoyl, carboxy or $R_6$ together with $R_5$ and the carbon atom to which they are attached represent a cyclopropyl group. In a further group of compounds $R_6$ represents hydrogen, a $C_{2-6}$ alkoxycarbonyl group, a $C_{1-4}$ alkylthio group, a $C_{1-4}$ alkylsulphinyl group, a $C_{1-4}$ alkylsulphonyl group, carbamoyl, carboxy or $R_6$ together with $R_5$ and the carbon atom to which they are attached represent a cyclopropyl group, especially hydrogen or a $C_{2-6}$

alkoxycarbonyl group. Most preferably $R_6$ represents hydrogen, chloro, bromo, acetyl, a $C_{2-4}$ alkoxycarbonyl group, methylthio, ethylthio, methylsulphinyl, methylsulphonyl, carbamoyl, or carboxy or $R_6$ together with $R_5$ and the carbon atom to which they are attached represents a cyclopropyl group. Particularly preferred are compounds in which $R_6$ represents hydrogen, methoxycarbonyl, ethoxycarbonyl, propoxycarbonyl or methylthio, especially hydrogen or a $C_{2-4}$ alkoxycarbonyl group, for example propoxycarbonyl (namely n-propoxycarbonyl and isopropoxycarbonyl).

Suitably $R_7$ represents hydrogen, halo, trifluoromethyl, a $C_{1-6}$ alkyl group, preferably a $C_{1-4}$ alkyl group (for example methyl, ethyl or propyl), a $C_{1-6}$ alkylthio group (for example methylthio or ethylthio) or a $C_{1-6}$ alkylsulphinyl group (for example methylsulphinyl or ethylsulphinyl). In preferred compounds of formula I, $R_7$ represents hydrogen, fluoro, chloro, trifluoromethyl, a $C_{1-6}$ alkylthio group or a $C_{1-6}$ alkylsulphinyl group. More preferably $R_7$ represents hydrogen, fluoro, chloro, trifluoromethyl, a $C_{1-4}$ alkylthio group or a $C_{1-4}$ alkylsulphinyl group. Most preferably $R_7$ represents hydrogen, fluoro, chloro, trifluoromethyl, methylthio or methylsulphinyl. Particularly preferred compounds of formula I are those in which $R_7$ represents fluoro, chloro, trifluoromethyl or methylthio, desirably fluoro, chloro or trifluoromethyl, especially fluoro or chloro.

In preferred compounds of formula I, $R_8$ represents hydrogen, fluoro or chloro, more preferably hydrogen or chloro, and most preferably hydrogen.

The substituents $R_9$ and $R_{10}$ may be located at any position on the benz ring, namely in position 6-, 7-, 8- and or 9- of the benz ring. Accordingly each of the substituents $R_9$ and $R_{10}$ specified herein are to be considered as named at each of these positions.

In preferred compounds of formula I, $R_9$ represents hydrogen.

Suitably $R_{10}$ represents hydrogen, fluoro, trifluoromethyl, hydroxy, nitro, a $C_{2-6}$ alkanoyloxy group (especially in position 8- or 9- of the benz ring), a $C_{1-6}$ alkyl group (especially in the 8- or 9-position of the benz ring) or a $C_{1-6}$ alkoxy group (especially in position 9- of the benz ring). As stated above $R_{10}$ may be located at the 6- position, especially for the substituents 6-fluoro, 6-hydroxy or 6-methoxy. The 7-position may also bear a substituent such as 7-fluoro, or 7-hydroxy. In preferred compounds of formula I, $R_{10}$ is located at position 8- or 9- of the benz ring and represents hydrogen, fluoro, trifluoromethyl, hydroxy, nitro, a $C_{2-5}$ alkanoyloxy group (for example acetoxy, propionyloxy, butanoyloxy or pentanoyloxy) or a $C_{1-4}$ alkyl group (for example methyl, ethyl or propyl). Preferably also $R_{10}$ represents a $C_{1-6}$ alkoxy group in position 9- of the benz ring (for example methoxy, ethoxy, propoxy or butoxy), more preferably a $C_{1-4}$ alkoxy group. More preferably $R_{10}$ represents hydrogen, fluoro, hydroxy or nitro and suitably also a $C_{2-6}$ alkanoyloxy group. Most preferably $R_{10}$ represents hydrogen, fluoro, hydroxy, a $C_{2-4}$ alkanoyloxy group, or $R_{10}$ represents a $C_{1-4}$ alkoxy group in position 9- of the benz ring. In particularly preferred compounds $R_{10}$ represents hydrogen, fluoro or a $C_{2-4}$ alkanoyloxy group in position 8- of the benz ring or hydroxy or a $C_{1-4}$ alkoxy group in position 9- of the benz ring, such as 9-ethoxy. Especially preferred are compounds in which $R_{10}$ represents hydrogen or 8-fluoro.

A preferred group of compounds according to the present invention are those wherein $R_1$ represents hydrogen or together with $R_2$ represents a bond; $R_2$ together with either one of $R_1$ and $R_3$ represents a bond; $R_3$ together with either one of $R_2$ and $R_4$ represents a bond; $R_4$ represents hydrogen or together with $R_3$ represents a bond; $R_5$ represents hydrogen; $R_6$ represents hydrogen, halo, a $C_{2-6}$ alkanoyl group, a $C_{2-6}$ alkoxycarbonyl group, a $C_{1-6}$ alkylthio group, a $C_{1-6}$ alkylsulphinyl group, a $C_{1-6}$ alkylsulphonyl group, carbamoyl, carboxy or $R_6$ together with $R_5$ and the carbon atom to which they are attached represent a cyclopropyl group; $R_7$ represents hydrogen, fluoro, chloro, trifluoromethyl, a $C_{1-6}$ alkylthio group or a $C_{1-6}$ alkylsulphinyl group; $R_8$ represents hydrogen or chloro; and $R_9$ and $R_{10}$ each represent fluoro or $R_9$ represents hydrogen and $R_{10}$ is a substituent in the 8- or 9- position of the benz ring and represents hydrogen, fluoro, trifluoromethyl, hydroxy, nitro, a $C_{2-6}$ alkanoyloxy group, a $C_{1-6}$ alkyl group, or $R_{10}$ represents a $C_{1-6}$ alkoxy group in position 9- of the benz ring.

A more preferred group of compounds of formula I are those in which $R_1$, $R_2$, $R_3$, $R_4$, $R_5$, $R_7$, $R_8$ and $R_9$ are described in the last preceeding paragraph and $R_6$ represents hydrogen, a $C_{2-6}$ alkoxycarbonyl group, a $C_{1-6}$ alkylthio group, a $C_{1-6}$ alkylsulphinyl group, a z$C_{1-6}$ alkylsulphonyl group, carbamoyl, carboxy or $R_6$ together with $R_5$ and the carbon atom to which they are attached represent a cyclopropyl group; and $R_{10}$ is a substituent in the 8- or 9- position of the benz ring and represents hydrogen, fluoro, trifluoromethyl, hydroxy, nitro, a $C_{1-6}$ alkyl group, or $R_{10}$ represents a $C_{1-6}$ alkoxy group in position 9- of the benz ring.

In compounds of formula II, the substituents $R_5$, $R_6$, $R_7$, $R_8$, $R_9$ and $R_{10}$ may be as hereinbefore defined. In a preferred group of compounds represented by formula II, $R_5$ represents hydrogen, $R_6$ represents hydrogen, methoxycarbonyl, ethoxycarbonyl, n-propoxycarbonyl, isopropoxycarbonyl or methylthio, $R_7$ represents fluoro, chloro, bromo, trifluoromethyl or methylthio, especially fluoro, chloro or

trifluoromethyl, $R_8$ represents hydrogen or chloro, $R_9$ represents hydrogen, and $R_{10}$ represents hydrogen, 9-hydroxy, 9-ethoxy, 8-propionyloxy or 8-fluoro.

In compounds of formula III, the substituents $R_5$, $R_6$, $R_7$, $R_8$, $R_9$ and $R_{10}$ may be as hereinbefore defined. In a preferred group of compounds represented by compounds of formula III, $R_5$ and $R_6$ each represent hydrogen, $R_7$ represents hydrogen or chloro, $R_8$ represents hydrogen or chloro and $R_9$ and $R_{10}$ each represent hydrogen.

Compounds of formula I may contain one or more chiral centres and exist in different optically active forms. When compounds of formula I contain one chiral centre the compounds exist in two enantiomeric forms and the present invention includes both enantiomers and mixtures of enantiomers. The enantiomers may be resolved by methods known to those skilled in the art, for example by high performance liquid chromatography on a chiral support, for example silica with a bound chiral ligand. For example, the following compounds of formula I exist as (R)- and (S)- enantiomers:-

Ethyl 2-(4-chlorophenyl)-α-methyl-3-oxo-2,3-dihydro[1]benzopyrano[4,3-c]pyrazole-4-acetate
2-(4-chlorophenyl)-4-methylsulphinylmethyl[1]benzopyrano[4,3-c]pyrazol-3(2H)-one
4-methyl-2-(4-trifluoromethylphenyl)-1,4-dihydro[1]benzopyrano[4,3-c]pyrazol-3(2H)-one
2-(3-chlorophenyl)-4-methyl-1,4-dihydro[1]benzopyrano[4,3-c]pyrazol-3(2H)-one
2-(3,4-dichlorophenyl)-4-methyl-1,4-dihydro[1]benzopyrano[4,3-c]pyrazol-3(2H)-one
2-(4-chlorophenyl)-4-methyl-1,4-dihydro[1]benzopyrano[4,3-c]pyrazol-3(2H)-one
2-(4-fluorophenyl)-4-methyl-1,4-dihydro[1]benzopyrano[4,3-c]pyrazol-3(2H)-one

When compounds of formula I contain more than one chiral centre, the compounds may exist in diastereoisomeric forms. The present invention includes each diastereoisomer and mixtures of the diastereoisomers. The diastereoisomers may be separated by methods known to those skilled in the art, for example by crystallisation followed by resolution.

The present invention also includes pharmaceutical compositions containing a therapeutically effective amount of a compound of formula I together with a pharmaceutically acceptable diluent or carrier.

In therapeutic use, the active compound may be administered orally, rectally, parenterally or topically, preferably orally or topically. Thus the therapeutic compositions of the present invention take the form of any of the known pharmaceutical compositions for oral, rectal, parenteral or topical administration. Pharmaceutically acceptable carriers suitable for use in such compositions are well known in the art of pharmacy. The compositions of the invention may contain 0.1-90% by weight of active compound. The compositions of the invention are generally prepared in unit dosage form. The excipients used in the preparation of these compositions are the excipients known in the pharmacist's art.

Compositions for oral administration are preferred compositions of the invention and these are known pharmaceutical forms for such administration, for example tablets, capsules, syrups and aqueous or oily suspensions. Tablets may be prepared by mixing the active compound with an inert diluent such as calcium phosphate in the presence of disintegrating agents, for example maize starch, and lubricating agents, for example magnesium stearate, and tableting the mixture by known methods. The tablets may be formulated in a manner known to those skilled in the art so as to give a sustained release of the compounds of the present invention. Such tablets may, if desired, be provided with enteric coatings by known methods, for example by the use of cellulose acetate phthalate. Similarly, capsules, for example hard or soft gelatin capsules, containing the active compound with or without added excipients, may be prepared by conventional means and, if desired, provided with enteric coatings in a known manner. The tablets and capsules may conveniently each contain 0.1 to 500 mg of the active compound. Other compositions for oral administration include, for example, aqueous suspensions containing the active compound in an aqueous medium in the presence of a non-toxic suspending agent such as sodium carboxymethylcellulose, and oily suspensions containing a compound of the present invention in a suitable vegetable oil, for example arachis oil.

Compositions for topical administration are also preferred compositions of the invention. The pharmaceutically active compound may be dispersed in a pharmaceutically acceptable cream, ointment or gel. A suitable cream may be prepared by incorporating the active compound in a topical vehicle such as petrolatum and/or light liquid paraffin, dispersed in an aqueous medium using surfactants. An ointment may be prepared by mixing the active compound with a topical vehicle such as a mineral oil, petrolatum and/or a wax e.g. paraffin wax or beeswax. A gel may be prepared by mixing the active compound with a topical vehicle comprising a gelling agent e.g. basified Carbomer BP, in the presence of water. Topically administrable compositions may also comprise a matrix in which the pharmaceutically active compounds of the present invention are dispersed so that the compounds are held in contact with the skin in order to administer the compounds transdermally. A suitable transdermal composition may be prepared by mixing the pharmaceutically active compound with a topical vehicle, such as described above, together with a

potential transdermal accelerant such as dimethyl sulphoxide or propylene glycol.

Compositions of the invention suitable for rectal administration are known pharmaceutical forms for such administration, for example suppositories with cocoa butter or polyethylene glycol bases.

Compositions of the invention suitable for parenteral administration are known pharmaceutical forms for such administration, for example sterile suspensions or sterile solutions in a suitable solvent.

In some formulations it may be beneficial to use the compounds of the present invention in the form of particles of very small size, for example as obtained by fluid energy milling.

In the compositions of the present invention the active compound may, if desired, be associated with other compatible pharmacologically active ingredients.

The compounds of formula I are immunomodulatory agents, generally manifested as immunosuppressants, but some compounds, in certain disease states, may exhibit immunostimulant activity. The compounds according to the invention are useful in the treatment of diseases resulting from an aberrant immune reaction. Thus the pharmaceutical compositions containing a therapeutically effective amount of a compound of formula I may be used to treat diseases with an immunological association for example tissue rejection, such as kidney rejection; autoimmune diseases, such as rheumatoid arthritis and systemic lupus erythematosus; cutaneous disorders, such as contact sensitivity, eczema and psoriasis; and neoplasia, such as melanoma.

In such treatment the amount of the compound of formula I administered per day will be such as to give a therapeutic effect and is generally in the range 0.1 to 2000 mg, preferably 1 to 500 mg.

Accordingly, in another aspect, the present invention also includes a method of treating diseases with an immunological association, comprising the administration of a therapeutically effective amount of a compound of formula I.

Processes for the preparation of compounds of formula I will now be described. These processes form a further aspect of the present invention.

Compounds of formula I which are represented by formula II may be prepared by oxidising compounds of formula I which are represented by formula III, for example by reaction with chloranil.

Compounds of formula I which are represented by formula II may be prepared by reacting a compound of formula IV.

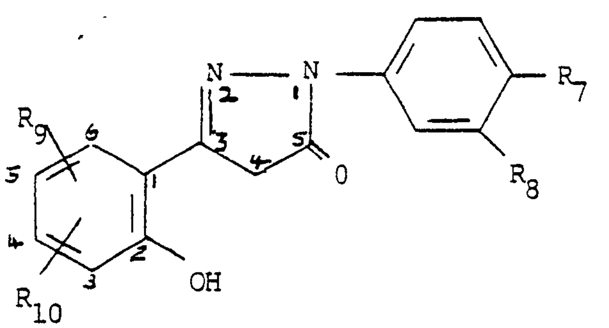

IV

or a tautomer thereof, with an ortho ester of formula V(a) or a thio ortho ester of formula V(b)

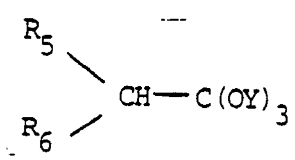

V(a)

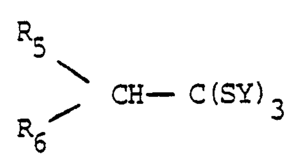

V(b)

in which Y represents a $C_{1-4}$ alkyl group or a benzyl group, for example by heating at 50-200° C. Alternatively compounds of formula I which are represented by formula II in which $R_6$ represents a $C_{2-6}$ alkanoyl group may be prepared by reacting a compound of formula IV with a 2,2,6-tri($C_{1-6}$alkyl)-4H-1,3-diox-5-en-4-one, in which the $C_{1-6}$alkyl groups may be the same or different, for example by heating at 50-200° C in an organic liquid.

Compounds of formula I which are represented by formula II may be prepared by reacting compounds of formula VI

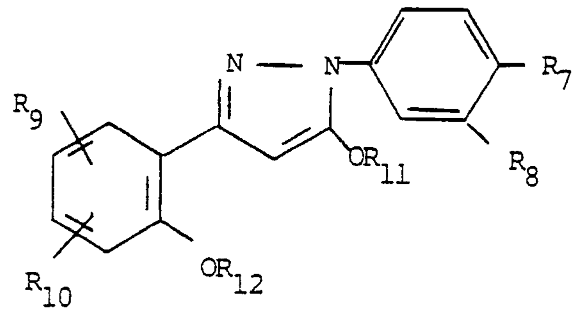

VI

in which $R_{11}$ represents hydrogen, or a tautomer thereof, or in which $R_{11}$ represents a group $COR_{13}$ wherein $R_{13}$ represents hydrogen, a benzyl group or a $C_{1-4}$ alkyl group and $R_{12}$ represents $COCHR_5R_6$ with a base e.g. piperidine in a suitable solvent, for example ethanol.

Compounds of formula I which are represented by formula III may be prepared by reducing compounds of formula I which are represented by formula II, for example by reaction with sodium borohydride.

Compounds of formula I which are represented by formula III may be prepared by reacting compounds of formula VII

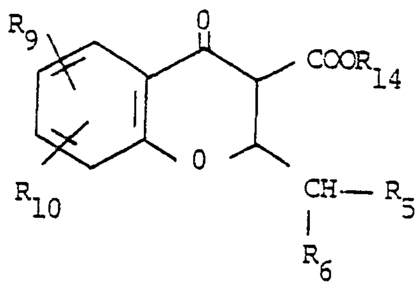

VII

in which $R_{14}$ represents a $C_{1-4}$ alkyl group or a benzyl group with a compound of formula VIII

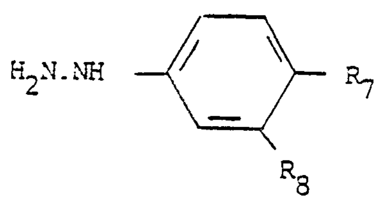

VIII

for example by heating at 50-200 °C in an organic liquid for example toluene.

Compounds of formula IV may be prepared by reacting compounds of formula IX

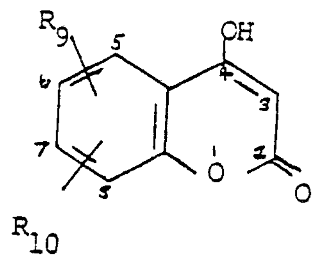

IX

with a compound of formula VIII, for example by heating at 50-200 °C in an organic liquid for example toluene. Preferably the compound of formula VII is used in excess of the stoichiometric amount.

Compounds of formula IV may be prepared by reacting compounds of formula X

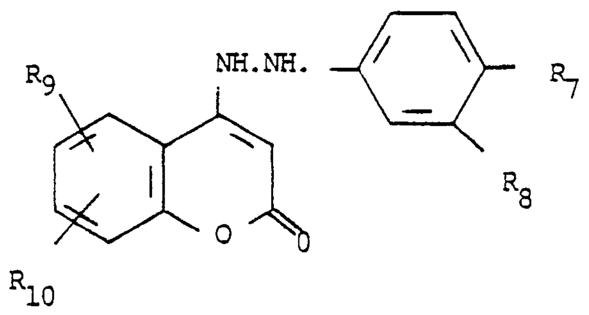

X

with an acid, for example hydrochloric acid, or with a base, for example a solution of sodium hydroxide.

Compounds of formula V(a) may be prepared for example by reacting compounds of formula $R_5R_6CHCN$ with an alcohol of formula YOH in the presence of an anhydrous acid, for example hydrogen chloride, to give compounds of formula $R_5R_6CHC(=NH)OY$ as their acid salts, e.g. hydrochloride salts, which are then reacted with further alcohol of formula YOH.

Compounds of formula V(b) may be prepared for example from compounds of formula $R_5R_6CHCOCl$ by reaction with thiols of formula YSH in the presence of a Lewis acid, for example zinc chloride.

Compounds of formula VI in which $R_{11}$ represents $COR_{13}$ and $R_{12}$ represents $COCHR_6R_5$ may be prepared by acylation of compounds of formula VI in which $R_{11}$ represents $COR_{13}$ and $R_{12}$ represents hydrogen, for example by reaction with an acid halide e.g. of formula $R_5R_6CHCOCl$.

Compounds of formula VI in which $R_{11}$ represents $COR_{13}$ and $R_{12}$ represents hydrogen may be prepared by the acylation of compounds of formula IV, for example by reaction with an acid anhydride of formula $(R_{13}CO)_2O$ in the presence of a salt (e.g. the sodium salt) of the corresponding acid.

Compounds of formula VI in which $R_{11}$ and $R_{12}$ are identical and represent $R_5R_6CHCO$ may be prepared by acylation of compounds of formula IV for example by using an acid anhydride of formula $(R_5R_6CHCO)_2O$ in the presence of a salt (e.g. the sodium salt) of the corresponding acid.

Compounds of formula VI in which $R_{12}$ represents $COCHR_6R_5$ and $R_{11}$ represents hydrogen, or tautomers thereof, may be prepared by reacting a compound of formula VI in which $R_{11}$ represents $COR_{13}$ and $R_{12}$ represents $COCHR_5R_6$ with a base e.g. piperidine in a suitable solvent e.g. ethanol.

Compounds of formula VII may be prepared by alkylating or cycloalkylating a compound of formula XI,

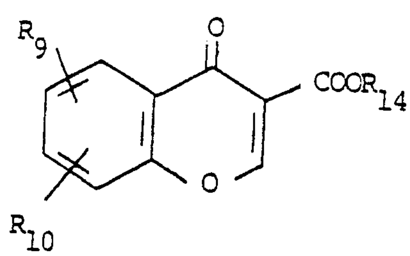

XI

for example by reaction with lithium dialkylcopper.

Compounds of formula VIII may be prepared by methods known to those skilled in the art.

Compounds of formula IX may be prepared by reacting compounds of formula XII

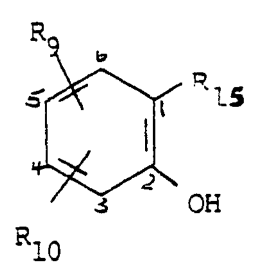

XII

in which $R_{15}$ represents hydrogen with malonic acid in the presence of an acid chloride e.g. phosphoryl chloride and a Lewis acid e.g. zinc chloride.

Compounds of formula IX may be prepared by reacting compounds of formula XII in which $R_{15}$

represents acetyl with a dialkyl carbonate of formula $(YO)_2CO$ in which Y represents a $C_{1-4}$ alkyl group or a benzyl group, for example, dimethyl carbonate, in the presence of a base, for example sodium hydride.

Compounds of formula X may be prepared by reacting compounds of formula IX with a hydrazine of formula VIII, for example by heating at 50-200 °C in a suitable solvent for example toluene. In cases where a mixture of compounds of formula IV and X are obtained, these compounds may be separated by virtue of their different solubilities in an organic liquid, for example dichloromethane.

Compounds of formula XI may be prepared by methods known to those skilled in the art.

Compounds of formula XII may be made by methods known to those skilled in the art.

Compounds of formula II in which $R_{10}$ represents hydroxy may be prepared by reacting compounds of formula II in which $R_{10}$ represents a $C_{1-6}$ alkoxy group with a Lewis acid, for example aluminium chloride. Compounds of formula II in which $R_{10}$ represents a $C_{2-6}$ alkoxycarbonyl group may be prepared by acylating compounds of formula II in which $R_{10}$ represents hydroxy, for example with the corresponding acid halide. Compounds of formula II in which $R_5$ and $R_6$ represent hydrogen may be prepared by decarboxylating compounds of formula II in which $R_5$ represents hydrogen and $R_6$ represents carboxyl, or by hydrolysing compounds of formula II in which $R_6$ represents a group which may be hydrolysed to a carboxyl group such as a $C_{2-6}$ alkoxycarbonyl group or carbamoyl, for example by reaction with sulphuric acid, followed by decarboxylation. Compounds of formula II in which $R_6$ represents a $C_{1-6}$ alkylsulphinyl group or a $C_{1-6}$ alkylsulphonyl group may be prepared by oxidation of compounds of formula II in which $R_6$ represents a $C_{1-6}$ alkylthio group with, for example, 3-chloroperoxybenzoic acid.

Compounds of formula IV may be prepared by reacting compounds of formula XIII

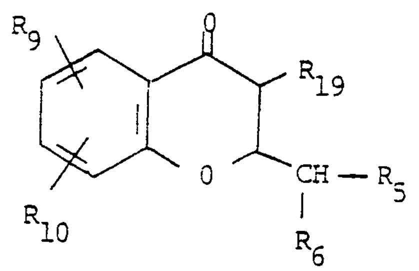

XIII

in which $R_{19}$ represents carbamoyl with compounds of formula VIII.

Compounds of formula XIII in which $R_{19}$ represents carbamoyl may be prepared by the hydrolysis of compounds of formula XIII in which $R_{19}$ represents cyano for example by reacting with methanol saturated with hydrogen chloride and then water.

Compounds of formula XIII in which $R_{19}$ represents cyano may be prepared by reaction of compounds of formula XIV

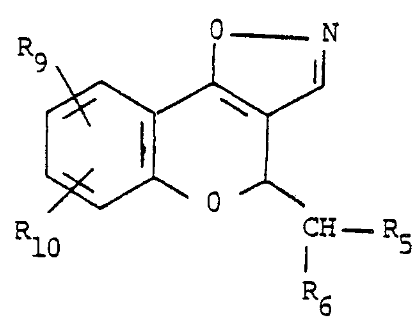

XIV

with a base, for example sodium ethoxide.

Compounds of formula XIV may be prepared from compounds of formula XV

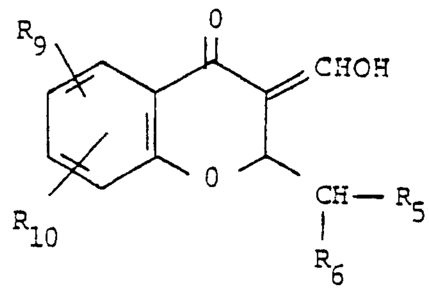

XV

by reaction with hydroxylamine hydrochloride.

Compounds of formula XV may be prepared from compounds of formula XIII in which $R_{19}$ represents hydrogen for example by heating with ethyl formate in the presence of a base, for example sodium ethoxide.

Some compounds of formula I, such as when $R_5$ and $R_6$ represents hydrogen, for example 2-(4-chlorophenyl)-4-methyl[1]benzopyrano[4,3-c]pyrazol-3(2H)-one, 2-(4-fluorophenyl)-4-methyl[1]benzopyrano-[4,3-c)pyrazol-3(2H)-one, or 2-(3,4-dichlorophenyl)-4-methyl[1]benzopyrano[4,3-c]pyrazol-3(2H)-one may rearrange in the presence of aqueous alkali and an organic liquid for example an alkanol such as isopropanol to form dimers of formula XVI

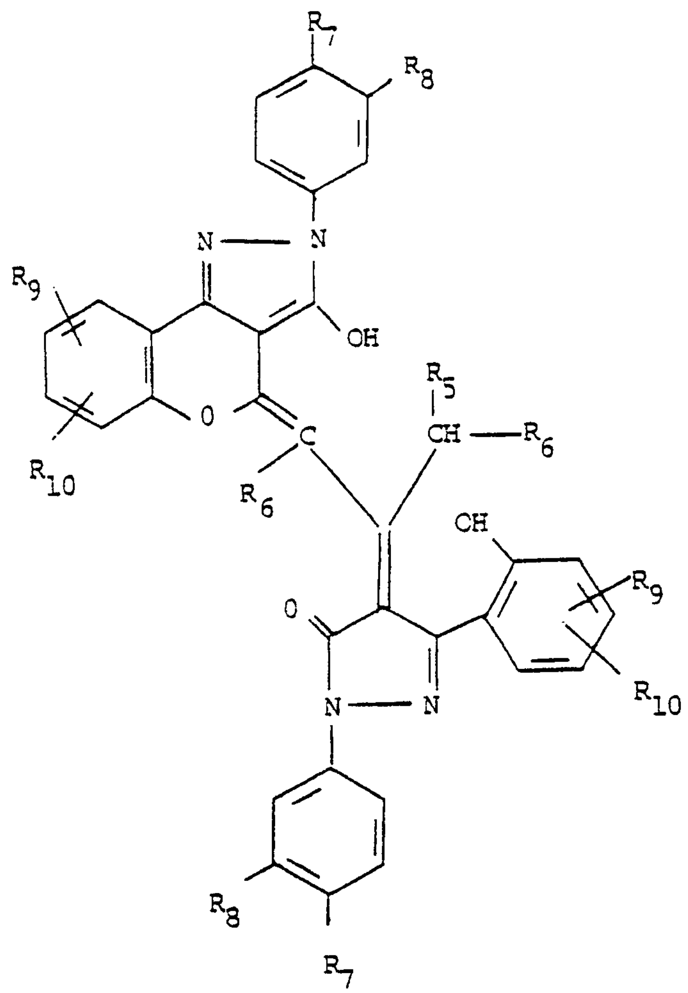

XVI

In the above three cases the dimers formed respectively are:-

1-(4-chlorophenyl)-4-{1-[2-(4-chlorophenyl)-3-hydroxy-2,4-dihydro[1]benzopyrano[4,3-c]pyrazol-4-ylidenemethyl]ethylidene}-3-(2-hydroxyphenyl)-2-pyrazolin-5-one.

1-(4-fluorophenyl)-4-{1-[2-(4-chlorophenyl)-3-hydroxy-2,4-dihydro[1]benzopyrano[4,3-c]pyrazol-4-ylidenemethyl]ethylidene}-3-(2-hydroxyphenyl)-2-pyrazolin-5-one.

1-(3,4-dichlorophenyl)-4-{1-[2-(3,4-dichlorophenyl)-3-hydroxy-2,4-dihydro[1]benzopyrano[4,3-c]pyrazol-4-ylidenemethyl]ethylidene}-3-(2-hydroxyphenyl)-2-pyrazolin-5-one.

Compounds of formula XVI are claimed as a further aspect of the invention.

Certain intermediate compounds of formulae II, III, IV, V, VI, VII, IX, X, XIII, XIV and XV are believed to be novel compounds. All novel compounds herein are claimed as a further aspect of the invention.

The invention is illustrated by the following non-limitative Examples.

The therapeutic activity of the compounds of the present invention has been demonstrated by a cutaneous hypersensitivity test (CH test) in which the compounds are administered parenterally to BALB/c mice. This test was carried out in the following way.

Female BALB/c mice, weight range 16-24 g, were used in groups of eight. The abdomen of each mouse was shaved and 20 µl of a solution of a sensitising agent, 5% w/v 4-ethoxymethylene-2-phenyl-2-oxazolin-5-one (oxazolone) in acetone:ethanol (1:1 by volume), was applied to the shaved area. Immediately after sensitisation, the test compound in one of the dosages listed below was injected intraperitoneally as a suspension in 1.5% v/v sorbitan esters, under the trade name Tween 80, in sterile water (100µl). 100 µl of the same suspension was injected likewise every 24 hours for a further 7 days. The dosages used were selected from the following values: 50, 30, 10, 3, 1, 0.3, 0.1, 0.03 or 0.01 mg/kg.

Two groups of eight BALB/c mice were used as a control simultaneously with each test in a similar manner to that described above except that no test compound was included in the daily injections.

On the seventh day after sensitisation, 10 µl of a solution of 1% w/v oxazolone in acetone: olive oil (either 1:1 or 3:1 by volume) was applied to one ear (the challenged ear) of each of the test mice and the control mice. After 24 hours the thickness of the challenged ear and the thickness of the non-challenged ear of the same animal were measured with an engineer's screw gauge micrometer. The difference in thickness between the challenged ear and the non-challenged ear in each animal is a measure of the response of that animal to oxazolone. A comparison between the response of mice treated with the test compound and mice treated with the control indicates the effectiveness of the test compound as an immunomodulatory agent. The compounds were considered to be active at a particular dose if a 20% or greater reduction in ear swelling, which was statistically significant ($p < 0.05$) according to Dunnett's test, between treated and control groups was obtained in two out of three CH tests, (or, where more than three tests have been carried out, a majority of the tests) at that dose (see for example Int. Arch. Allergy, 38, p246-259 (1970)).

Each of the compounds of formula I illustrated in Table A below was active at 50 mg/kg in two out of three tests at 50 mg/kg unless indicated otherwise (see Notes following the Table). The minimum effective dose (ie the lowest dose of those used at which the compound is active as defined above) for each compound is given in Table A. The Example (Ex) number or numbers listed adjacent to each compound indicates the process or processes illustrating the preparation of that compound in the Examples.

Table A

| Ex | Compound Name | Minimum Effective Dose (mg/kg) |
|---|---|---|
| 36 | 2-(3,4-dichlorophenyl)-4-methyl[1]-benzopyrano[4,3-*c*]pyrazol-3(2*H*)-one | 0.3 |
| 37 | 2-(4-fluorophenyl)-4-methyl[1]benzopyrano[4,3-*c*]pyrazol-3(2*H*)-one | 1 |
| 38 | 2-(3-chlorophenyl)-4-methyl[1]benzopyrano[4,3-*c*]pyrazol-3(2*H*)-one | 3 |
| 39 | 2-(4-methoxyphenyl)-4-methyl[1]benzo-pyrano[4,3-*c*]pyrazol-3(2*H*)-one | 50 |
| 40 | 2-(4-chlorophenyl)-4-ethyl[1]benzo-pyrano[4,3-*c*]pyrazol-3(2*H*)-one | 50 |
| 41 | ethyl 2-(4-chlorophenyl)-3-oxo-2,3-dihydro[1]benzopyrano[4,3-*c*]pyrazole-4-acetate | 3 |
| 42 | 4-methyl-2-phenyl[1]benzopyrano-[4,3-*c*]pyrazol-3(2*H*)-one | 50 (a) |
| 43/96 | 4-methyl-2-(4-methylphenyl)[1]benzopyrano[4,3-*c*]pyrazol-3(2*H*)-one | 10 |
| 44 | 2-(4-bromophenyl)-4-methyl[1]benzo-pyrano[4,3-*c*]pyrazol-3(2*H*)-one | 1 |
| 45 | 2-(3-chloro-4-fluorophenyl)-4-methyl-[1]benzopyrano[4,3-*c*]pyrazol-3(2*H*)-one | 0.3 |

Table A (Continued)

| | | |
|---|---|---|
| 46 | 2-(3-chloro-4-methylphenyl)-4-methyl-[1]benzopyrano[4,3-_c_]pyrazol-3(2_H_)-one | 3 |
| 47 | 4-methyl-2-(4-trifluoromethylphenyl)-[1]benzopyrano[4,3-_c_]pyrazol-3(2_H_)-one | 1 |
| 48 | 2-(4-chlorophenyl)-7-fluoro-4-methyl-[1]benzopyrano[4,3-_c_]pyrazol-3(2_H_)-one | 50 |
| 49 | 8-chloro-2-(4-chlorophenyl)-4-methyl-[1]benzopyrano[4,3-_c_]pyrazol-3(2_H_)-one | 50 |
| 50 | 2-(4-chlorophenyl)-4,8-dimethyl[1]-benzopyrano[4,3-_c_]pyrazol-3(2_H_)-one | 50 |
| 51 | 2-(4-chlorophenyl)-9-methoxy-4-methyl-[1]benzopyrano[4,3-_c_]pyrazol-3(2_H_)-one | 50 |
| 52 | 2-(4-chlorophenyl)-4,9-dimethyl[1]-benzopyrano[4,3-_c_]pyrazol-3(2_H_)-one | 50 |
| 53 | ethyl 2-(4-fluorophenyl)-3-oxo-2,3-dihydro[1]benzopyrano[4,3-_c_]pyrazole-4-acetate | 3 |
| 54 | 2-(4-chlorophenyl)-8-fluoro-4-methyl-[1]benzopyrano[4,3-_c_]pyrazol-3(2_H_)-one | 0.1 |
| 55 | 7-chloro-2-(4-chlorophenyl)-4-methyl-[1]benzopyrano[4,3-_c_]pyrazol-3(2_H_)-one | 50 |
| 56 | 2-(4-chlorophenyl)-8-hydroxy-4-methyl-[1]benzopyrano[4,3-_c_]pyrazol-3(2_H_)-one | 50 |

Table A (Continued)

| | | |
|---|---|---|
| 57 | 4-methyl-2-(4-methylthiophenyl)[1]-benzopyrano[4,3-*c*]pyrazol-3(2*H*)-one | 3 |
| 58 | 2-(4-chlorophenyl)-9-ethoxy-4-methyl-[1]benzopyrano[4,3-*c*]pyrazol-3(2*H*)-one | 3 |
| 59 | 2-(4-chlorophenyl)-4-methyl-8-nitro-[1]benzopyrano[4,3-*c*]pyrazol-3(2*H*)-one | 50 |
| 60 | 2-(4-chlorophenyl)-8-ethyl-4-methyl-[1]benzopyrano[4,3-*c*]pyrazol-3(2*H*)-one | 50 |
| 61 | 6-chloro-4-methyl-2-phenyl[1]benzo-pyrano[4,3-*c*]pyrazol-3(2*H*)-one | 50 |
| 62 | 2-(4-chlorophenyl)-6-fluoro-4-methyl-[1]benzopyrano[4,3-*c*]pyrazol-3(2*H*)-one | 50 |
| 63 | 2-(4-chlorophenyl)-6,8-difluoro-4-methyl[1]benzopyrano[4,3-*c*]pyrazol-3(2*H*)-one | 50 |
| 64 | 4-bromomethyl-2-(4-chlorophenyl)[1]-benzopyrano[4,3-*c*]pyrazol-3(2*H*)-one | 50 |
| 65 | 4-chloromethyl-2-(4-chlorophenyl)[1]-benzopyrano[4,3-*c*]pyrazol-3(2*H*)-one | 3 |
| 66 | 2-(4-chlorophenyl)-6-methoxy-4-methyl-[1]benzopyrano[4,3-*c*]pyrazol-3(2*H*)-one | 50 |
| 67 | 4-methyl-2-(4-methylsulphinylphenyl)-[1]benzopyrano[4,3-*c*]pyrazol-3(2*H*)-one | 50 |

Table A (Continued)

| | | |
|---|---|---|
| 69 | 2-(4-chlorophenyl)-4-methylthiomethyl-[1]benzopyrano[4,3-*c*]pyrazol-3(2*H*)-one | 3 |
| 74 | 2-(4-chlorophenyl)-3-oxo-2,3-dihydro-[1]benzopyrano[4,3-*c*]pyrazole-4-acetamide | 50 |
| 75 | ethyl 2-(4-chlorophenyl)-α-methyl-3-oxo-2,3-dihydro[1]benzopyrano[4,3-*c*]-pyrazole-4-acetate | 50 |
| 76 | propyl 2-(4-chlorophenyl)-3-oxo-2,3-dihydro[1]benzopyrano[4,3-*c*]pyrazole-4-acetate | 0.3 |
| 77 | isopropyl 2-(4-chlorophenyl)-3-oxo-2,3-dihydro[1]benzopyrano[4,3-*c*]pyrazole-4-acetate | 0.3 |
| 78 | 2-(4-chlorophenyl)-4-cyclopropyl[1]-benzopyrano[4,3-*c*]pyrazol-3(2*H*)-one | 50 |
| 79 | methyl 2-(4-chlorophenyl)-3-oxo-2,3-dihydro[1]benzopyrano[4,3-*c*]pyrazole-4-acetate | 3 |
| 80 | 2-(4-chlorophenyl)-4-ethylthiomethyl-[1]benzopyrano[4,3-*c*]pyrazol-3(2*H*)-one | 50 |
| 81 | 2-(4-chlorophenyl)-4-methyl-3-oxo-2,3-dihydro[1]benzopyrano[4,3-*c*]pyrazol-9-yl acetate | 50 |

Table A (Continued)

| | | | |
|---|---|---|---|
| 82 | 2-(4-chlorophenyl)-4-methyl-3-oxo-2,3-dihydro[1]benzopyrano[4,3-*c*]pyrazol-8-yl acetate | 50 | (b) |
| 83 | 2-(4-chlorophenyl)-4-(2-oxopropyl)[1]-benzopyrano[4,3-*c*]pyrazol-3(2*H*)-one | 3 | |
| 84 | 2-(4-chlorophenyl)-9-hydroxy-4-methyl-[1]benzopyrano[4,3-*c*]pyrazol-3(2*H*)-one | 3 | |
| 85 | 2-(4-chlorophenyl)-7-hydroxy-4-methyl-[1]benzopyrano[4,3-*c*]pyrazol-3(2*H*)-one | 50 | |
| 86 | 2-(4-chlorophenyl)-6-hydroxy-4-methyl-[1]benzopyrano[4,3-*c*]pyrazol-3(2*H*)-one | 50 | |
| 87 | 2-(4-chlorophenyl)-4-methylsulphonylmethyl[1]benzopyrano[4,3-*c*]pyrazol-3(2*H*)-one | 50 | |
| 88 | 2-(4-chlorophenyl)-4-methylsulphinylmethyl[1]benzopyrano[4,3-*c*]pyrazol-3(2*H*)-one | 50 | |
| 89 | 2-(4-chlorophenyl)-4-methyl-3-oxo-2,3-dihydro[1]benzopyrano[4,3-*c*]pyrazol-8-yl propionate | 3 | |
| 90 | 2-(4-chlorophenyl)-4-methyl-3-oxo-2,3-dihydro[1]benzopyrano[4,3-*c*]pyrazol-8-yl butyrate | 3 | |
| 91/ 92/ 93/ 95 | 2-(4-chlorophenyl)-4-methyl[1]benzopyrano[4,3-*c*]pyrazol-3(2*H*)-one | 0.1 | |

Table A (Continued)

| | | | |
|---|---|---|---|
| 97 | 4-methyl-2-(4-trifluoromethylphenyl)-1,4-dihydro[1]benzopyrano[4,3-c]-pyrazol-3(2H)-one | 3 | |
| 98 | 2-(3-chlorophenyl)-4-methyl-1,4-dihydro[1]benzopyrano[4,3-c]pyrazol-3(2H)-one | 3 | |
| 99 | 2-(3,4-dichlorophenyl)-4-methyl-1,4-dihydro[1]benzopyrano[4,3-c]pyrazol-3(2H)-one | 50 | |
| 100 | 2-(4-chlorophenyl)-4-methyl-1,4-dihydro[1]benzopyrano[4,3-c]pyrazol-3(2H)-one | 3 | (c) |
| 101 | 2-(4-fluorophenyl)-4-methyl-1,4-dihydro[1]benzopyrano[4,3-c]pyrazol-3(2H)-one | 50 | |
| 102 | 2-(4-chlorophenyl)-3-oxo-2,3-dihydro[1]benzopyrano[4,3-c]pyrazole-4-acetic acid | 50 | |
| 103 | 1-(4-chlorophenyl)-4-{1-[2-(4-chlorophenyl)-3-hydroxy-2,4-dihydro[1]benzopyrano[4,3-c]pyrazol-4-ylidenemethyl]-ethylidene}-3-(2-hydroxyphenyl)-2-pyrazolin-5-one. | 3 | |
| 104 | 1-(3,4-dichlorophenyl)-4-{1-[2-(3,4-dichlorophenyl)-3-hydroxy-2,4-dihydro[1]benzopyrano[4,3-c]pyrazol-4-ylidenemethyl]-ethylidene}-3-(2-hydroxyphenyl)-2-pyrazolin-5-one. | 50 | |

## Notes

a): Activity in three out of five tests at 50 mg/kg.

b): Activity in one test at 50 mg/kg and activity in one test at 18 mg/kg

c): Activity in a single test at 50 mg/kg and activity in both tests at 30 mg/kg.

The compounds of the present invention also show activity in a variety of other in-vivo screens, which show the utility of the compounds as immunomodulants, particularly in suppressing the immune response. Administration of the compounds has been carried out orally, parenterally or topically. Some compounds have been found to be active in a test which determines their effects on humoral immunity by assaying the sera collected at the end of the oxazolone induced cutaneous hypersensitivity test described above (CH test) for changes in the amount of anti-oxazolone antibody produced, and a Graft versus Host test similar to that used by Smith S R, Terminelli C, Kipilman C T and Smith Y., J. Immunopharmacology 1981;3(2),133-170.

In the Examples parts and percentages are by weight and compositions of mixed solvents are given by volume. Characterisation was by elemental analysis and one or more of the following spectroscopic techniques: nuclear magnetic resonance, infra-red and mass spectroscopy.

### Example 1

Powdered aluminium chloride (97.4 g) was added in portions to 2-fluorophenyl acetate (74.9 g) with swirling. The reaction mixture was slowly heated to 150°C and kept at this temperature for 2 hours. The reaction mixture was cooled to 40°C and the solid formed was added to iced water (300 ml) containing concentrated hydrochloric acid (5 ml). The reaction mixture was steam distilled and the distillate cooled and extracted with ethyl acetate. The extracts were dried and evaporated to give 3′-fluoro-2′-hydroxyacetophenone, m.p. 75-77°C.

### Example 2

A mixture of 5′-fluoro-2′-hydroxyacetophenone (10 g) in dry toluene (130 ml) was added dropwise over 20 minutes to a stirred suspension of sodium hydride (6.17 g; 60% dispersion in mineral oil) in dry toluene (130 ml) which was boiling under reflux under nitrogen. After boiling for a further 10 minutes heating was continued while a solution of diethyl carbonate (15.7 ml) in dry toluene (130 ml) was added dropwise over 25 minutes. This mixture was stirred and heated under reflux for 4 hours. On cooling, the reaction mixture was poured on to iced 2M hydrochloric acid (700 ml). The solid obtained was collected by filtration and then dissolved in 4M aqueous sodium hydroxide (325 ml). This solution was washed with ether and then acidified with 5M hydrochloric acid. The solid obtained was collected by filtration, washed with water and dried to give 6-fluoro-4-hydroxycoumarin, m.p. 250-251°C.

### Examples 3-6

In a similar way to that described in Example 2, compounds of formula IX were prepared from compounds of formula XII was summarized in Table 1 below.

TABLE 1

| Example | XII | | Amount of Reactants | | | | Reflux Time (hours) | m.p. of IX (°C) | Notes |
|---|---|---|---|---|---|---|---|---|---|
| | $R_9$ | $R_{10}$ | XII (g) | Sodium hydride (g) | Diethyl carbonate (g) | Total Toluene (ml) | | | |
| 3 | 3-F | H | 12.0 | 8.45 | 18.4 | 450 | 24 | 246-248 | (1)(2)(3) |
| 4 | 5-$C_2H_5$ | H | 40.0 | 22.0 | 58.5 | 450 | 20 | 190-192 | |
| 5 | 3-$OCH_3$ | H | 14.3 | 7.77 | 20.3 | 450 | 24 | 244-246 | (4) |
| 6 | 3-F | 5-F | 14.0 | 7.32 | 19.5 | 480 | 4 | 236-240 | |

m.p. = melting point

NOTES

(1): Recrystallised from industrial methylated spirit.
(2): 50% dispersion of sodium hydride in mineral oil used.
(3): Starting material prepared in Example 1.
(4): Recrystallised from propan-2-ol.

In each starting compound of formula XII, $R_{15}$ represents $COCH_3$. $R_9$ and $R_{10}$ are as indicated in Table 1. The compounds of formula IX prepared in Examples 3-6 were as follows:

Example 3 8-Fluoro-4-hydroxycoumarin.
Example 4 6-Ethyl-4-hydroxycoumarin.
Example 5 4-Hydroxy-8-methoxycoumarin.
Example 6 6,8-Difluoro-4-hydroxycoumarin.

Example 7

a) 2-Fluoro-6-methoxybenzonitrile (34 g) was added in portions over 10-15 minutes to a stirred suspension of methyl magnesium iodide prepared from magnesium (6.5 g) and methyl iodide (38.4 g) in toluene (300 ml) under nitrogen. The mixture was stirred and heated under reflux for 18 hours then cooled to ambient temperature and 3M hydrochloric acid (200 ml) added. This mixture was stirred and heated under reflux for 18 hours then cooled to ambient temperature and the organic layer separated. The aqueous layer was extracted with ether and the combined ether and toluene extracts were washed with water, dried and evaporated to give an oil (41.1 g). A solution of this oil (36 g) in dichloromethane (250 ml) was treated dropwise with a solution of boron tribromide (35.7 g) in dichloromethane (60 ml) at -70 to -80 °C over 25 minutes. The mixture was stirred at -80° for 10 minutes then allowed to warm up to 0 °C. Water (100 ml) was added, keeping the temperature below 10 °C. The organic phase was separated, washed, dried, and evaporated to give an oil which was distilled under reduced pressure to give 2′-fluoro-6′-hydroxyacetophenone, b.p. 170-172 °C (0.2 mm Hg).

b) 2′-Fluoro-6′-hydroxyacetophenone (30 g) was added dropwise over 30 minutes to a stirred suspension of sodium hydride (17.1g , 60% dispersion in mineral oil) in dry toluene (400 ml) which was boiling under reflux under nitrogen. After boiling for a further 20 minutes heating was continued while diethyl carbonate (50.8 g) was added dropwise over 10 minutes. The resulting mixture was stirred and heated under reflux for 20 hours. The reaction mixture was cooled to ambient temperature and water (150 ml) added dropwise. After separation, the aqueous phase was washed with ether and then acidified with concentrated hydrochloric acid. The precipitate was collected by filtration and washed with water to give, after drying and recrystallisation from ethyl acetate, 5-ethoxy-4-hydroxycoumarin, m.p. 110-111 °C.

c) A mixture of 5-ethoxy-4-hydroxycoumarin (4.2 g) and 4-chlorophenylhydrazine (3.7 g) in xylene (20 ml) was heated under reflux for 6 hours with removal of the water produced in the reaction. The mixture was cooled to ambient temperature and the xylene removed under reduced pressure. The residue was heated under reflux with methanol (50 ml) and concentrated hydrochloric acid (50 ml) for 2.5 hours, then cooled to ambient temperature. The mixture was evaporated to dryness under reduced pressure and the residue

triturated with water to give a solid which was dried to give 1-(4-chlorophenyl)-3-(6-ethoxy-2-hydroxyphenyl)-2-pyrazolin-5-one.

Example 8

a) A stirred mixture of 4-hydroxycoumarin (17.2 g) and 3,4-dichlorophenylhydrazine (18.8 g) in dry toluene (174 ml) was heated under reflux for 4.25 hours with removal of the water produced in the reaction. After allowing to cool, the mixture was kept at ambient temperature for 16 hours and then filtered to give a solid, being a mixture of 1-(3,4-dichlorophenyl)-3- (2-hydroxyphenyl)-2-pyrazolin-5-one and 4-[2-(3,4-dichlorophenyl)hydrazino]coumarin. The solid was heated with dichloromethane (approximately 500 ml). After hot filtration and cooling the liquors, a solid product was deposited being 1-(3,4-dichlorophenyl)-3-(2-hydroxyphenyl)-2-pyrazolin-5-one, m.p. 196-200 °C.

Examples 9-32

In a similar wat to that described in Example 8, compounds of formula IV were prepared by reacting compounds of formula VIII with compounds of formula IX, as summarized in Table 2 below. The substituents $R_7$ and $R_8$ of compounds VIII and the substituents $R_9$ and $R_{10}$ of compounds IX are given in Table 2.

TABLE 2

| | VIII | | IX | | Amount of Reactants | | | | | |
|---|---|---|---|---|---|---|---|---|---|---|
| Ex | $R_7$ | $R_8$ | $R_9$ | $R_{10}$ | IX (g) | VIII (g) | Toluene (ml) | Reflux Time (hours) | m.p. of IV (°C) | Notes |
| 9 | F | H | H | H | 15.0 | 11.7 | 153 | 4.2 | 170-173 | |
| 10 | H | Cl | H | H | 5.03 | 4.86 | 50 | 2.0 | 146-148 | (1) (2) |
| 11 | $OCH_3$ | H | H | H | 23.1 | 21.7 | 500 | 2.0 | 133-135 | (1) (2) |
| 12 | H | H | H | H | 5.62 | 4.12 | 50 | 2.0 | 119-121 | (3) |
| 13 | $CH_3$ | H | H | H | 13.4 | 11.1 | 100 | 2.0 | 180-182 | (1) (2) |
| 14 | Br | H | H | H | 11.6 | 20.1 | 122 | 2.2 | 195-198 | |
| 15 | F | Cl | H | H | 6.5 | 9.7 | 100 | 3.5 | 191-193 | |
| 16 | $CH_3$ | Cl | H | H | 2.0 | 2.7 | 10 | 4.0 | 157-159 | (4) |
| 17 | $CF_3$ | H | H | H | 1.97 | 3.0 | 15 | 4.0 | 174-177 | (4) |
| 18 | Cl | H | H | 7-F | 1.0 | 1.18 | 15 | 4.0 | 172-174 | (4) |
| 19 | Cl | H | H | 6-Cl | 1.96 | 2.14 | 15 | 4 | 217-221 | |
| 20 | Cl | H | H | 6-$CH_3$ | 17.6 | 21.4 | 150 | 4 | 232-234 | |

Ex = Example m.p. = melting point

TABLE 2 continued

| | VIII | | IX | | Amount of Reactants | | | | | |
|---|---|---|---|---|---|---|---|---|---|---|
| Ex | $R_7$ | $R_8$ | $R_9$ | $R_{10}$ | IX (g) | VIII (g) | Toluene (ml) | Reflux Time (hours) | m.p. of IV (°C) | Notes |
| 21 | Cl | H | H | 5-$CH_3$ | 5.0 | 6.07 | 55 | 3 | 183-185 | (5) (6) |
| 22 | Cl | H | H | 6-F | 6.0 | 7.83 | 60 | 2.5 | 180-183 | (7) |
| 23 | Cl | H | H | 7-Cl | 2.10 | 2.25 | 35 | 3 | 198-199 | (4) |
| 24 | Cl | H | H | 6-$OCH_3$ | 9.20 | 15.2 | 82 | 7.5 | 197-203 | (8) |
| 25 | $SCH_3$ | H | H | H | 8.46 | 12.1 | 100 | 1 | 138-140 | (9) |
| 26 | Cl | H | H | 7-$OCH_3$ | 10.0 | 11.0 | 140 | 5 | 189-190 | (4) |
| 27 | Cl | H | H | 6-$NO_2$ | 7.3 | 10.5 | 100 | 8 | 220 | (10) |
| 28 | Cl | H | H | 6-$C_2H_5$ | 5.5 | 4.9 | 120 | 4 | 192-194 | (11) |
| 29 | H | H | H | 8-Cl | 6.74 | 5.56 | 200 | 24 | 208-210 | (12) |
| 30 | Cl | H | H | 8-F | 4.5 | 5.35 | 100 | 24 | 219-221 | (2) |
| 31 | Cl | H | H | 8-$OCH_3$ | 3.25 | 3.62 | 100 | 24 | 204-206 | (12) |
| 32 | Cl | H | 6-F | 8-F | 4.4 | 4.85 | 100 | 1 | 229-232 | (4) |

Ex = Example m.p. = melting point

NOTES

(1): Reflux solution allowed to cool and solvent evaporated to yield solid prior to heating with dichloromethane.

(2): Recrystallisation from acetonitrile.

(3): Recrystallisation from cyclohexane.

(4): Dichloromethane extracts were evaporated to dryness.

(5): Starting material was a mixture of 5-methyl-4-hydroxycoumarin and 7-methyl-4-hydroxycoumarin.

(6): Desired compound IV obtained by fractional crystallisation of a mixture of isomers from dichloromethane.

(7): Reaction mixture allowed to cool and filtered. Filtrate concentrated until product crystallised.

(8): Reaction mixture filtered to give IV.

(9): Dichloromethane extracts were evaporated and residue triturated with ether.

(10): p-Toluenesulphonic acid used as catalyst.

(11): Reaction mixture allowed to cool and solvent evaporated. Residue recrystallised from ethyl acetate.

(12): Crude product recrystallised from toluene instead of dichloromethane.

The compounds of formula IV prepared in Examples 9-32 were as follows:-

| Example | Compound |
|---|---|
| 9 | 1-(4-fluorophenyl)-3-(2-hydroxyphenyl)-2-pyrazolin-5-one. |
| 10 | 1-(3-chlorophenyl)-3-(2-hydroxyphenyl)-2-pyrazolin-5-one. |
| 11 | 3-(2-hydroxyphenyl)-1-(4-methoxyphenyl)-2-pyrazolin-5-one. |
| 12 | 3-(2-hydroxyphenyl)-1-phenyl-2-pyrazolin-5-one. |
| 13 | 3-(2-hydroxyphenyl)-1-(4-methylphenyl)-2-pyrazolin-5-one. |
| 14 | 1-(4-bromophenyl)-3-(2-hydroxyphenyl)-2-pyrazolin-5-one. |
| 15 | 1-(3-chloro-4-fluorophenyl)-3-(2-hydroxyphenyl)-2-pyrazolin-5-one. |
| 16 | 1-(3-chloro-4-methylphenyl)-3-(2-hydroxyphenyl)-2-pyrazolin-5-one. |
| 17 | 3-(2-hydroxyphenyl)-1-(4-trifluoromethylphenyl)-2-pyrazolin-5-one. |
| 18 | 1-(4-chlorophenyl)-3-(4-fluoro-2-hydroxyphenyl)-2-pyrazolin-5-one. |
| 19 | 1-(4-chlorophenyl)-3-(5-chloro-2-hydroxyphenyl)-2-pyrazolin-5-one. |
| 20 | 1-(4-chlorophenyl)-3-(2-hydroxy-5-methylphenyl)-2-pyrazolin-5-one. |
| 21 | 1-(4-chlorophenyl)-3-(2-hydroxy-6-methylphenyl)-2-pyrazolin-5-one. |
| 22 | 1-(4-chlorophenyl)-3-(5-fluoro-2-hydroxyphenyl)-2-pyrazolin-5-one. |
| 23 | 3-(4-chloro-2-hydroxyphenyl)-1-(4-chlorophenyl)-2-pyrazolin-5-one. |
| 24 | 1-(4-chlorophenyl)-3-(2-hydroxy-5-methoxyphenyl)-2-pyrazolin-5-one. |

| Example | Compound |
|---|---|
| 25 | 3-(2-hydroxyphenyl)-1-(4-methylthiophenyl)-2-pyrazolin-5-one. |
| 26 | 1-(4-chlorophenyl)-3-(2-hydroxy-4-methoxyphenyl)-2-pyrazolin-5-one. |
| 27 | 1-(4-chlorophenyl)-3-(2-hydroxy-5-nitrophenyl)-2-pyrazolin-5-one. |
| 28 | 1-(4-chlorophenyl)-3-(5-ethyl-2-hydroxyphenyl)-2-pyrazolin-5-one. |
| 29 | 3-(3-chloro-2-hydroxyphenyl)-1-phenyl-2-pyrazolin-5-one. |
| 30 | 1-(4-chlorophenyl)-3-(3-fluoro-2-hydroxyphenyl)-2-pyrazolin-5-one. |
| 31 | 1-(4-chlorophenyl)-3-(2-hydroxy-3-methoxyphenyl)-2-pyrazolin-5-one. |
| 32 | 1-(4-chlorophenyl)-3-(3,5-difluoro-2-hydroxyphenyl)-2-pyrazolin-5-one. |

Example 33

a) A mixture of 4-hydroxycoumarin (10.8 g) and 4-chlorophenylhydrazine (9.5 g) in dry toluene (110 ml) was stirred and heated under reflux for 1.5 hours with removal of the water produced in the reaction. The mixture was left standing at ambient temperature overnight and the solid obtained was collected by filtration. This was boiled in dichloromethane (approximately 200 ml) and hot filtered to give, after recrystallisation from industrial methylated spirit, 4-[2-(4-chlorophenyl)hydrazino]coumarin, m.p. 226-229 °C.

b) A mixture of 4-[2-(4-chlorophenyl)hydrazino]coumarin (2.86 g), ethanol (250 ml) and 5M hydrochloric acid (2 ml) was stirred and boiled under reflux for 21.5 hours. On cooling, the reaction mixture was filtered. The filtrate was evaporated and the residue was partitioned between dichloromethane and water. The dichloromethane extracts were combined, dried and concentrated to give 1-(4-chlorophenyl)-3-(2-hydroxyphenyl)-2-pyrazolin-5-one, m.p. 182-187 °C.

Example 34

a) A stirred mixture of 4-hydroxy-5-methoxycoumarin (6.5 g), 4-chlorophenylhydrazine (7.3 g) and dry toluene (66 ml) was heated under reflux with removal of the water formed in the reaction. On cooling, the solid obtained was collected by filtration to give 4-[2-(4-chlorophenyl)hydrazino]-5-methoxycoumarin, m.p. 206-209 °C.

b) A mixture of 4-[2-(4-chlorophenyl)hydrazino]-5-methoxycoumarin (1.58 g), 5M aqueous sodium hydroxide (1 ml) and industrial methylated spirit (100 ml) was boiled under reflux for 4 hours. On cooling, the mixture was filtered. The filtrate was evaporated to dryness and the residue was partitioned between dichloromethane and water. The dichloromethane layer was separated off, dried and concentrated to give, after filtration, 1-(4-chlorophenyl)-3-(2-hydroxy-6-methoxyphenyl)-2-pyrazolin-5-one, m.p. 185-188 °C.

Example 35

A mixture of 3-(2-hydroxyphenyl)-1-(4-methylthiophenyl)-2-pyrazolin-5-one (2.3 g) (Example 25) 3-chloroperoxy-benzoic acid (1.7 g ; 80%) and dichloromethane (172 ml) was stirred at ambient temperature for 66 hours. The mixture was washed with saturated sodium bicarbonate solution, then water, then dried and evaporated. The residue was recrystallised from toluene / petroleum ether (b.p. 62-68 °C) to give 3-(2-hydroxyphenyl)-1-(4-methylsulphinylphenyl)-2-pyrazolin-5-one, m.p. 76-80 °C.

Example 36

A stirred mixture of 1-(3,4-dichlorophenyl)-3-(2-hydroxyphenyl)-2-pyrazolin-5-one (2.0 g) (Example 8) and triethyl orthoacetate (3.41 ml) was heated at 130-135 °C for 10 minutes. The mixture was allowed to cool to ambient temperature and kept at this temperature for 16 hours. It was then triturated with ether and the solid product collected and dried to give 2-(3,4-dichlorophenyl)-4-methyl[1]benzopyrano[4,3-*c*]-pyrazol-3(2*H*)-one, m.p. 207-211 °C.

Examples 37-68

In a similar way to that described in Example 36, compounds of formula II were prepared by reacting compounds of formula IV with compounds of formula V(a) as summarized in Table 3. The substituents $R_7$, $R_8$, $R_9$ and $R_{10}$ of compounds IV are given in Table 3. In compounds of formula V(a), $R_{18}$ represents $R_5R_6CH$ and Y represents $C_2H_5$ unless otherwise stated. In the Example Number column, the number given in brackets indicates the Example Number illustrating the preparation of compounds of formula IV. The amount of compounds V(a) is given in ml unless otherwise stated.

TABLE 3

| Example | IV | | | | Va | Amount of Reactants | | | | |
|---|---|---|---|---|---|---|---|---|---|---|
| | $R_7$ | $R_8$ | $R_9$ | $R_{10}$ | $R_{18}$ | IV (g) | Va (ml) | Heating Time (mins) | m.p. of II (°C) | Notes |
| 37(9) | F | H | H | H | $CH_3$ | 3.0 | 6.0 | 10 | 183-187 | |
| 38(10) | H | Cl | H | H | $CH_3$ | 3.52 | 6.75 | 60 | 171-173 | (1) |
| 39(11) | $OCH_3$ | H | H | H | $CH_3$ | 6.0 | 11.7 | 60 | 177-179 | (2) |
| 40(91a) | Cl | H | H | H | $C_2H_5$ | 4.29 | 9.0 | 10 | 167-171 | |
| 41(91a) | Cl | H | H | H | $CH_2COOC_2H_5$ | 2.87 | 7.02 | 10 | 159-161 | |
| 42(12) | H | H | H | H | $CH_3$ | 2.52 | 5.49 | 60 | 163-165 | (1) |
| 43(13) | $CH_3$ | H | H | H | $CH_3$ | 6.76 | 14.0 | 60 | 162-164 | (3) |
| 44(14) | Br | H | H | H | $CH_3$ | 4.0 | 6.6 | 20 | 196-199 | |
| 45(15) | F | Cl | H | H | $CH_3$ | 3.1 | 11.2 | 20 | 204-207 | |

TABLE 3 continued

| | IV | | | | Va | Amount of Reactants | | | | |
|---|---|---|---|---|---|---|---|---|---|---|
| Example | $R_7$ | $R_8$ | $R_9$ | $R_{10}$ | $R_{18}$ | IV (g) | Va (ml) | Heating Time (mins) | m.p. of II (°C) | Notes |
| 46(16) | $CH_3$ | Cl | H | H | $CH_3$ | 1.0 | 1.79 | 15 | 188-190 | (5) |
| 47(17) | $CF_3$ | H | H | H | $CH_3$ | 0.5 | 0.86 | 15 | 199-200 | (5) |
| 48(18) | Cl | H | H | 4-F | $CH_3$ | 0.5 | 0.91 | 15 | 194-198 | (5) |
| 49(19) | Cl | H | H | 5-Cl | $CH_3$ | 0.65 | 1.1 | 10 | 190-192 | |
| 50(20) | Cl | H | H | 5-$CH_3$ | $CH_3$ | 3.00 | 8.2 | 10 | 195-197 | |
| 51(34) | Cl | H | H | 6-$OCH_3$ | $CH_3$ | 1.10 | 1.9 | 25 | 226-230 | |
| 52(21) | Cl | H | H | 6-$CH_3$ | $CH_3$ | 0.50 | 0.9 | 15 | 208-210 | |
| 53(9) | F | H | H | H | $CH_2COOC_2H_5$ | 1.20 | 3.1g | 15 | 152-154 | |
| 54(22) | Cl | H | H | 5-F | $CH_3$ | 1.52 | 2.75 | 10 | 187-189 | |
| 55(23) | Cl | H | H | 4-Cl | $CH_3$ | 1.00 | 1.7 | 15 | 230-231 | |
| 56(82a) | Cl | H | H | 5-OH | $CH_3$ | 1.40 | 5.6 | 10 | 315-319 | |
| 57(25) | $SCH_3$ | H | H | H | $CH_3$ | 3.00 | 5.5 | 10 | 174-176 | |
| 58(7c) | Cl | H | H | 6-$OC_2H_5$ | $CH_3$ | 2.8 | 4.9g | 35 | 216-218 | |
| 59(27) | Cl | H | H | 5-$NO_2$ | $CH_3$ | 7.0 | 15.0 | 90 | 257-258 | (6) |
| 60(28) | Cl | H | H | 5-$C_2H_5$ | $CH_3$ | 3.7 | 5.8g | 30 | 193-194 | |

TABLE 3 continued

| Example | IV | | | | Va | Amount of Reactants | | Heating Time (mins) | m.p. of II (°C) | Notes |
|---|---|---|---|---|---|---|---|---|---|---|
| | $R_7$ | $R_8$ | $R_9$ | $R_{10}$ | $R_{18}$ | IV (g) | Va (ml) | | | |
| 61(29) | H | H | H | 3-Cl | $CH_3$ | 3.0 | 10.0 | 30 | 209-211 | (4)(7) |
| 62(30) | Cl | H | H | 3-F | $CH_3$ | 2.0 | 8.0 | 30 | 232-234 | (4)(7) |
| 63(32) | Cl | H | 3-F | 5-F | $CH_3$ | 1.05 | 1.8 | 10 | 214-216 | |
| 64(91a) | Cl | H | H | H | $CH_2Br$ | 8.0 | 20.0g | 50 | 207-208 | (8) |
| 65(91a) | Cl | H | H | H | $CH_2Cl$ | 1.43 | 2.31g | 10 | 237-240 | (9) |
| 66(31) | Cl | H | H | 3-$OCH_3$ | $CH_3$ | 3.0 | 5.2 | 30 | 243-245 | (4) |
| 67(35) | $SOCH_3$ | H | H | H | $CH_3$ | 0.7 | 1.2 | 20 | 228-230 | |
| 68(26) | Cl | H | H | 4-$OCH_3$ | $CH_3$ | 2.30 | 3.91 | 15 | 201-204 | |

NOTES

(1): Recrystallisation from isopropanol.

(2): Recrystallisation from acetonitrile.

(3): Recrystallisation from propanol.

(4): Reactants heated at 135-145°C.

(5): Reactants heated at 125°C.

(6): Purified by dissolving in dichloromethane and diluting with industrial methylated spirit, filtering and evaporating the filtrate.

(7): Recrystallisation from ethyl acetate.

(8): Toluene added with reactants and mixture boiled under reflux.

(9): Y = $CH_3$ in formula V(a).

Example 69

a) STAGE A A solution of methylthioacetonitrile (100 g) and methanol (47 ml) in dry ether (644 ml) was saturated with hydrogen chloride at 0-5° C. The mixture was allowed to warm to ambient temperature during 16 hours. The resulting solid product was collected by filtration, washed and dried to give methyl methylthioacetamidate hydrochloride as a sticky solid.

b) STAGE B A mixture of the methyl methylthioacetamidate hydrochloride (from 69a) and methanol (551 ml) was stirred at 35-45° C for three hours and then left at ambient temperature for 72 hours. The mixture was then filtered and the filtrate evaporated to give an oil containing a little solid which was removed by filtration through cotton wool giving trimethyl ortho(methylthio)acetate as an oil, b.p. 96-104° C (5 mm Hg).

c) STAGE C Trimethyl ortho(methylthio)acetate (5 ml) was added to 1-(4-chlorophenyl)-3-(2-hydroxyphenyl)-2-pyrazolin-5-one (2.87 g), (Example 91a), and the mixture was heated at 135-145° C for 10 minutes, triturated with ether (10 ml) and then filtered. The residue was washed with excess water followed by excess ether to give 2-(4-chlorophenyl)-4-methylthiomethyl[1]benzopyrano[4,3-c]pyrazol-3(2H)-one, m.p. 215-217° C.

Examples 70-73

In a similar way to that described in Example 69 STAGES A and B, compounds of formula V(a) were prepared as summarized in Table 4.

TABLE 4

STAGE A - PREPARATION OF IMIDATE HYDROCHLORIDES

| Example | Nitrile | Amount (g) | Alcohol | Amount | Amount of Ether (ml) |
|---|---|---|---|---|---|
| 70 | $NCCH_2CONH_2$ | 79.8 | $C_2H_5OH$ | 110 ml | 536(2) |
| 71 | $NCCH(CH_3)CO_2C_2H_5$ | 30.5 | $C_2H_5OH$ | 14 ml | 134(3)(4) |
| 72 | $NCCH_2CO_2C_3H_7$(n) | 30.5 | (n)$C_3H_7OH$ | 18.5 ml | 134(3) |
| 73 | $NCCH_2CO_2C_3H_7$(i) | 15.0 | $CH_3OH$ | 4.2 g | 70(1) |

STAGE B - PREPARATION OF ORTHO ESTERS

| Example | Alcohol | Amount of alcohol | Temperature °C | Heating Time (hours) | Boiling Point of Va |
|---|---|---|---|---|---|
| 70 | $C_2H_5OH$ | 750 ml (1) | 40-45 | 22 | decomposes on heating |
| 71 | $C_2H_5OH$ | 180 ml (1) | 45-50 | 24 | 130-142°C (30 mm Hg) |
| 72 | (n)$C_3H_7OH$ | 180 ml (1) | 45-50 | 24 | 165-175°C (5 mm Hg) |
| 73 | $CH_3OH$ | 53 ml (5) | 30-40 | 16 | 180-190°C (10 mm Hg) |

NOTES

(1): Ether added at the end of the reaction.

(2): Dry dioxan (110 ml) added to increase solubility and ether (250 ml) added at the end of the reaction.

(3): Reaction mixture evaporated to leave the imidate.

(4): Crystallised from ether.

(5): Ether (290 ml) added at the start of the reaction.

The compounds prepared in Table 4 Stage B were as follows:-

Example 70 Triethyl ortho(carbamoyl)acetate

Example 71 Triethyl ortho(2-ethoxycarbonyl)propionate

Example 72 Tripropyl ortho(propoxycarbonyl)acetate

Example 73 Trimethyl ortho(isopropoxycarbonyl)acetate

Examples 74-77

In a similar way to that described in Example 69 STAGE C, compounds of formula II were prepared from compounds of formula V(a) and IV as summarized in Table 5 below. In compounds of formula V(a), $R_{18}$ represents $R_6R_5CH$ and in compounds of formula IV $R_7$, $R_8$, $R_9$ and $R_{10}$ are as given in Table 5.

TABLE 5

| Ex | IV | | | | Va | IV Amount (g) | Va Amount (g) | Heating Time (mins) | m.p. of II (°C) | Notes |
|---|---|---|---|---|---|---|---|---|---|---|
| | $R_7$ | $R_8$ | $R_9$ | $R_{10}$ | $R_{18}$ | | | | | |
| 74 | Cl | H | H | H | $CH_2CONH_2$ | 2.87 | 6.15 | 10 | 253-256 | (1)(4) |
| 75 | Cl | H | H | H | $CH(CH_3)COOC_2H_5$ | 2.87 | 7.44 | 30 | 130-134 | (2)(4) |
| 76 | Cl | H | H | H | $CH_2COOC_3H_7(n)$ | 2.87 | 8.7 | 40 | 138-140 | (2)(3) |
| 77 | Cl | H | H | H | $CH_2COOC_3H_7(i)$ | 0.46 | 2.0 | 15 | 124-126 | (1) |

Ex = Example m.p. = melting Point

NOTES

(1): Reactants heated at 125-135 °C.

(2): Reaction mixture cooled and triturated with industrial methylated spirit. Product collected by filtration.

(3): Y = n-$C_3H_7$ in formula V(a).

(4): Y = $C_2H_5$ in formula V(a).

Example 78

a) A mixture of 1-(4-chlorophenyl)-3-(2-hydroxyphenyl)-2-pyrazolin-5-one (1.43 g) (Example 91a) and acetic anhydride (7.5 ml) was treated with anhydrous sodium acetate (0.41 g) and stirred for 2.5 hours at ambient temperature. The reaction mixture was poured into a mixture of water and petrol (b.p. 62-68°, 9:1).

The solid product collected was washed with water and petrol (b.p. 62-68°) and then dried to give 1-(4-chlorophenyl)-3-(2-hydroxyphenyl)-5-pyrazolyl acetate, m.p. 108-110° C.

b) Cyclopropanecarbonyl chloride (2 ml) was added dropwise to a stirred solution of 1-(4-chlorophenyl)-3-(2-hydroxyphenyl)-5-pyrazolyl acetate (3.29 g) in dry tetrahydrofuran (50 ml) and triethylamine (3 ml) at 0 to 5° C and the mixture stirred, the temperature being allowed to rise to ambient, for sixteen hours. The mixture was then poured into water (10 volumes) and extracted with ethyl acetate. The extracts were washed with water, dried and evaporated to give 2-[5-acetoxy-1-(4-chlorphenyl)-3-pyrazolyl]phenyl cyclopropanecarboxylate as an oily product.

c) A mixture of the 2-[5-acetoxy-1-(4-chlorophenyl)-3-pyrazolyl]phenyl cyclopropanecarboxylate (from 78b), piperidine (1 ml) and absolute ethanol (30 ml) was heated under reflux for 2 hours. The solid product obtained was collected, washed with ethanol and dried to give 2-(4-chlorophenyl)-4-cyclopropyl[1]benzopyrano-[4,3-c]pyrazol-3(2H)-one, m.p. 240-243° C.

Example 79

a) In a similar manner to Example 78a, a mixture of 1-(4-chlorophenyl)-3-(2-hydroxyphenyl)-2-pyrazolin-5-one (1.9 g) (Example 91a), butyric anhydride (10 ml) and sodium butyrate (0.73 g) gave 1-(4-chlorophenyl)-3-(2-hydroxyphenyl)-5-pyrazolyl butyrate, m.p. 119-122° C.

b) Methyl malonyl chloride (1.2 ml) was added dropwise to a stirred mixture of 1-(4-chlorophenyl)-3-(2-hydroxyphenyl)-5-pyrazolyl butyrate (3.57 g) in dry tetrahydrofuran (50 ml) and triethylamine (1.5 ml) at 0-5° C. The mixture was stirred at ambient temperature for 24 hours and then more methyl malonyl chloride (0.6 ml) and triethylamine (0.75 ml) were added. The reaction mixture was stirred at ambient temperature for 24 hours and then treated in a similar manner to Example 78b to give an oil, from which unreacted starting material was removed by trituration with ether.

c) This product was boiled under reflux in dry toluene (25 ml) and piperidine (1 ml) for 1.5 hours. On cooling, the product was collected by filtration to give methyl 2-(4-chlorophenyl)-3-oxo-2,3-dihydro[1]-benzopyrano[4,3-c]pyrazole-4-acetate, m.p. 170-172° C.

Example 80

Ethylthioacetyl chloride (1 ml) was added dropwise to a stirred mixture of 1-(4-chlorophenyl)-3-(2-hydroxyphenyl)-5-pyrazolyl butyrate (3.57 g) (Example 79a) in dry tetrahydrofuran (50 ml) and triethylamine (1.5 ml) at 0-5° C. The mixture was stirred at ambient temperature for 2 days and then cooled to 0-5° C and more triethylamine (0.75 ml) and ethylthioacetyl chloride (0.25 ml) added. The mixture was stirred at ambient temperature for 16 hours and then treated in a similar manner to Example 78b to give a gum which was boiled under reflux in absolute ethanol (20 ml) containing piperidine (1 ml) for 35 minutes. On cooling the mixture was filtered to give 2-(4-chlorophenyl)-4-ethylthiomethyl[1]benzopyrano[4,3-c]pyrazol-3(2H)-one, m.p. 167° C.

Example 81

a) A mixture of 1-(4-chlorophenyl)-3-(2-hydroxy-6-methoxyphenyl)-2-pyrazolin-5-one (1.52 g) (Example 34), aluminium chloride (2.54 g) and dry xylene (16 ml) was stirred at 100-110° C for 35 minutes. On cooling, the xylene was decanted off and 2M hydrochloric acid (32.6 ml) and ice were added to the residual brown gum. The solid produced was collected by filtration, washed and dried, and recrystallised from aqueous industrial methylated spirit to give 1-(4-chlorophenyl)-3-(2,6-dihydroxyphenyl)-2-pyrazolin-5-one, m.p. 290-293° C.

b) 1-(4-Chlorophenyl)-3-(2,6-dihydroxyphenyl)-2-pyrazolin-5-one, (1.36 g), acetic anhydride (6.75 ml) and sodium acetate (0.37 g) were stirred at ambient temperature for 3 hours. More sodium acetate (0.74 g) was added and the stirring continued for 16 hours. The mixture was added to a mixture of water and petrol (b.p. 60-80° , 9:1). The solid produced was collected by filtration and after drying was stirred in a mixture of dry tetrahydrofuran (26 ml) and triethylamine (1.42 ml) in an ice-water bath while acetyl chloride (0.74 ml) was added dropwise. The mixture was stirred for 3.3 hours and then added to water (10 volumes). The solid obtained was filtered off and dried to give 1-(4-chlorophenyl)-3-(2,6-diacetoxyphenyl)-5-pyrazolyl acetate m.p. 140-142° C.

c) A mixture of 1-(4-chlorophenyl)-3-(2,6-diacetoxyphenyl)-5-pyrazolyl acetate (1.2 g), piperidine (0.28 ml) and ethanol (5.5 ml) was boiled under reflux for 30 minutes. On cooling, the solid obtained was

collected by filtration to give 2-(4-chlorophenyl)-4-methyl-3-oxo-2,3-dihydro[1]benzopyrano[4,3-c]pyrazol-9-yl acetate, m.p. 233-236 °C.

Example 82

a) 1-(4-Chlorophenyl)-3-(2-hydroxy-5-methoxyphenyl)-2-pyrazolin-5-one (5.5 g) (Example 24), aluminium chloride (9.35 g) and dry xylene (66 ml) were stirred and heated at 100 °C for 1 hour and then treated in a similar manner to Example 81a to give, after recrystallisation from methanol, 1-(4-chlorophenyl)-3-(2,5-dihydroxyphenyl)-2-pyrazolin-5-one, m.p. 220-225 °C.

b) 1-(4-Chlorophenyl)-3-(2,5-dihydroxyphenyl)-2-pyrazolin-5-one (0.6 g), acetic anhydride (3 ml) and anhydrous sodium acetate (0.5 g) were boiled and stirred under reflux for 2 hours. The mixture was poured into water and the product extracted into ethyl acetate. Evaporation of the ethyl acetate gave an oil which was boiled under reflux in absolute ethanol (4 ml) and piperidine (0.2 ml) for 3 hours. The solid obtained on cooling was collected by filtration to give 2-(4-chlorophenyl)-4-methyl-3-oxo-2,3-dihydro[1]benzo[4,3-c]-pyrazol-8-yl acetate, m.p. 192-194 °C.

Example 83

A mixture of 2,2,6-trimethyl-4H-1,3-diox-5-en-4-one (2.85 g) in xylene (15 ml) was added dropwise over 15 minutes to a stirred mixture of 1-(4-chlorophenyl)-3-(2-hydroxyphenyl)-2-pyrazolin-5-one (3.8 g) (Example 91b) in xylene (20 ml) at 130-135 °C allowing any low boiling material to distil off. The mixture was stirred and heated for 30 minutes and then more 2,2,6-trimethyl-4H-1,3-diox-5-en-4-one (1.4 g) in xylene (7.5 ml) was added over 10 minutes. The mixture was stirred and heated for 1 hour and then the xylene was removed under reduced pressure. The solid residue obtained was recrystallised from propan-2-ol to give 2-(4-chlorophenyl)-4-(2-oxopropyl) [1]benzopyrano[4,3-c]pyrazol-3(2H)-one, m.p. 167-168 °C.

Example 84

A mixture of 2-(4-chlorophenyl)-9-methoxy-4-methyl[1]benzopyrano[4,3-c]pyrazol-3(2H)-one (0.50 g) (Example 51) and aluminium chloride (0.78 g) in dry xylene (4.8 ml) was placed in a preheated oil bath at 100-110 °C for 35 minutes. On cooling, 2M hydrochloric acid (10 ml) and ice were added to the reaction mixture. The yellow solid obtained was filtered to give 2-(4-chlorophenyl)-9-hydroxy-4-methyl[1]-benzopyrano[4,3-c]pyrazol-3(2H)-one, m.p. 213-215 °C.

Example 85

In a similar manner to Example 84 2-(4-chlorophenyl)-7-methoxy-4-methyl[1]benzopyrano[4,3-c]pyrazol-3(2H)-one (1.0 g) (Example 68), dry xylene (12 ml) and aluminium chloride (1.7 g) were heated at 100 °C for 2 hours to give 2-(4-chlorophenyl)-7-hydroxy-4-methyl[1]benzopyrano[4,3-c]pyrazol-3(2H)-one, m.p. >300 °C.

Example 86

In a similar manner to Example 84 2-(4-chlorophenyl)-6-methoxy-4-methyl[1]benzopyrano[4,3-c]pyrazol-3(2H)-one (1.0 g) (Example 66), aluminium chloride (1.7 g) and dry xylene (12 ml) were heated at 100 °C for 3 hours to give, after recrystallisation from N,N-dimethylformamide, 2-(4-chlorophenyl)-6-hydroxy-4-methyl-[1]benzopyrano[4,3-c]pyrazol-3(2H)-one, m.p. >300 °C.

Example 87

A mixture of 2-(4-chlorophenyl)-4-methylthiomethyl[1]benzopyrano[4,3-c]pyrazol-3(2H)-one (1.5 g) (Example 69c) and 3-chloroperoxybenzoic acid (80%, 1.8 g) in dichloromethane (265 ml) was stirred at ambient temperature for 48 hours. More 3-chloroperoxybenzoic acid (80%, 0.2 g) in dichloromethane (25 ml) was added and the mixture stirred for 48 hours. The reaction mixture was washed with saturated sodium

bicarbonate solution, then water, then dried and concentrated. The solid product was collected by filtration to give 2-(4-chlorophenyl)-4-methylsulphonylmethyl[1]benzopyrano[4,3-c]pyrazol-3(2H)-one, m.p. 240-243 °C.

Example 88

A mixture of 2-(4-chlorophenyl)-4-methylthiomethyl[1]benzopyrano[4,3-c]pyrazol-3(2H)-one (1.5 g) (Example 69c) and 3-chloroperoxybenzoic acid (80%, 0.9 g) in dichloromethane (172 ml) was stirred at ambient temperature for 66 hours. Filtration gave 2-(4-chlorophenyl)-4-methylsulphinylmethyl[1]-benzopyrano[4,3-c]pyrazol-3(2H)-one, m.p. 193-194 °C.

Example 89

A mixture of 2-(4-chlorophenyl)-8-hydroxy-4-methyl[1]benzopyrano[4,3-c]pyrazol-3-(2H)-one (1.5 g) (Example 56), dry pyridine (45 ml) and propionyl chloride (0.9 ml) was stirred in an ice-bath and then allowed to warm up to ambient temperature over 16 hours. The mixture was poured on to water and extracted with ethyl acetate. The combined extracts were washed several times with water, dried and evaporated giving a solid which was triturated with ether and then recrystallised from industrial methylated spirit to give (2-(4-chlorophenyl)-4-methyl-3-oxo-2,3-dihydro[1]benzopyrano[4,3-c]pyrazol-8-yl propionate m.p. 191-193 °C.

Example 90

Butyryl chloride (1.64 ml) was added dropwise to a stirred mixture of 2-(4-chlorophenyl)-8-hydroxy-4-methyl[1]benzopyrano[4,3-c)pyrazol-3(2H)-one (2.0 g) (Example 56), dichloromethane (50 ml) and triethylamine (2.2 ml) at 0-5 °C. The mixture was stirred at ambient temperature for 20 hours and then washed with water, dried and evaporated under reduced pressure. The residue was triturated with ether and filtered to give 2-(4-chlorophenyl)-4-methyl-3-oxo-2,3-dihydro[1]benzopyrano[4,3-c]pyrazol-8-yl butyrate, m.p. 170-173 °C.

Example 91

a) A stirred mixture of 4-hydroxycoumarin (14.3 g) and 4-chlorophenylhydrazine (18.9 g) in dry toluene (150 ml) was heated under reflux for 2.5 hours with removal of the water produced in the reaction. The reaction mixture was allowed to cool to ambient temperature, then filtered and the solid product collected to give 1-(4-chlorophenyl)-3-(2-hydroxyphenyl)-2-pyrazolin-5-one, m.p. 183-185 °C.

b) A stirred mixture of 1-(4-chlorophenyl)-3-(2-hydroxyphenyl)-2-pyrazolin-5-one (1.43 g) and triethyl orthoacetate (2.74 ml) was heated at 130-135 °C for 10 minutes. The reaction mixture was allowed to cool to ambient temperature, triturated with ether (10 ml) and the solid product collected and dried to give 2-(4-chlorophenyl)-4-methyl[1]benzopyrano[4,3-c]pyrazol-3(2H)-one, m.p. 204-206 °C.

Example 92

A stirred mixture of 1-(4-chlorophenyl)-3-(2-hydroxyphenyl)-2-pyrazolin-5-one (0.29 g) (Example 91a), sodium acetate (0.25 g) and acetic anhydride (1.5 ml) was heated under reflux for 0.5 hours. The reaction mixture was cooled to ambient temperature, diluted with water and extracted with ether. The extracts were washed with water, dried and evaporated to give a semi-crystalline gum. A mixture of this gum and piperidine (0.08 ml) in ethanol (2 ml) was heated under reflux for 1.5 hours then cooled, diluted with water and acidified with concentrated hydrochloric acid. The solid product collected by filtration was 2-(4-chlorophenyl)-4-methyl[1]benzopyrano[4,3-c]pyrazol-3(2H)-one, m.p. 202-205 °C.

Example 93

2-(4-Chlorophenyl)-3-oxo-2,3-dihydro[1]benzopyrano[4,3-c]pyrazol-4-acetamide (0.8 g) (Example 74), and sulphuric acid (75%, 25 ml) were stirred and heated at 100-120° C for 9 hours. The mixture was poured on to ice (10 volumes) and the solid obtained collected by filtration to give 2-(4-chlorophenyl)-4-methyl[1]-benzopyrano[4,3-c]pyrazol-3(2H)-one, m.p. 204-206° C.

Example 94

a) Sodium (2.24 g) was dissolved in A.R. methanol (24 ml) and treated with a solution of ethyl formate (8.1 ml) in A.R. methanol (52 ml). The mixture was stirred at 0-5° C while 6-fluoro-2-methyl-4-chromanone (8.78 g) was added in portions over 5 minutes. This mixture was stirred for 1 hour then left at ambient temperature overnight. The solvent was removed by evaporation and the residue partitioned between toluene and water after adding 5M sodium hydroxide solution to pH > 10. The separated aqueous layer was acidified with 2M hydrochloric acid to give 6-fluoro-3-hydroxymethylene-2-methyl-4-chromanone, m.p. 68-73° C.

b) A mixture of 6-fluoro-3-hydroxymethylene-2-methyl-4-chromanone (1.8 g), hydroxylamine hydrochloride (0.49 g) and glacial acetic acid (41 ml) was boiled under reflux with stirring for 10 minutes. Water (40 ml) was added and the product collected by filtration to give 8-fluoro-4-methyl-4H-[1]-benzopyrano[3,4-d]isoxazole, m.p. 84-87° C.

c) A stirred suspension of 8-fluoro-4-methyl-4H[1]benzopyrano[3,4-d]isoxazole (0.125 g) in absolute ethanol (2 ml) was treated dropwise with a solution of sodium (0.017 g) in ethanol (0.4 ml). The resultant solution was stirred for 3 hours then acidified with 5M hydrochloric acid and diluted with water. The solid obtained on cooling was collected by filtration and dried to give 6-fluoro-2-methyl-4-oxo-3-chromancarbonitrile, m.p. 125-128° C.

d) A mixture of 6-fluoro-2-methyl-4-oxo-3-chromancarbonitrile (1.1 g) and A.R. methanol (50 ml) was saturated with hydrogen chloride at 0-5° C and left standing for 66 hours. The mixture was evaporated and the residue partitioned between water and ethyl acetate. The combined ethyl acetate extracts were dried and concentrated to give a solid which was collected by filtration giving 6-fluoro-2-methyl-4-oxo-3-chromancarboxamide, m.p. 161-165° C.

e) A mixture of 6-fluoro-2-methyl-4-oxo-3-chromancarboxamide (0.467 g) and 4-chlorophenylhydrazine (0.298 g) in glacial acetic acid (1 ml) was heated at 110-115° C with stirring for 1 hour with more 4-chlorophenylhydrazine (0.075 g) being added after 45 minutes. The mixture was cooled, diluted with ether, washed with water, dried and evaporated. The residue was purified by preparative thin layer chromatography on silica using toluene/glacial acetic acid (9:1) as the mobile phase to give 1-(4-chlorophenyl)-3-(5-fluoro-2-hydroxyphenyl)-2-pyrazolin-5-one, m.p. 178-182° C.

Example 95

Methyllithium (74 ml of a 1.4 molar solution in ether) was added over 25 minutes to a stirred suspension of dimethyl sulphide-copper (I) bromide complex (21.1 g) in dry ether (800 ml) under nitrogen at -10 to -15° C. The resulting mixture was cooled to -40° C, a solution of methyl 4-oxo-4H-chromen-3-carboxylate (7.0 g) in dry ether (100 ml) added dropwise and the mixture stirred at -40° C for 30 minutes. Saturated aqueous ammonium chloride solution (100 ml) was added dropwise keeping the temperature between -40 and -20° C and then the mixture was allowed to warm up to ambient temperature and extracted with ethyl acetate. The combined extracts were washed, dried and evaporated to give a yellow oil (8.6 g). This oil was dissolved in toluene (250 ml) containing 4-chlorophenylhydrazine (4.3 g) and the mixture boiled under reflux under nitrogen for 3 hours with removal of the water formed. The mixture was cooled to ambient temperature and p-chloroanil (8.3 g) added. The mixture was heated and stirred under reflux under nitrogen for 2 hours then cooled to ambient temperature and left to stand for 16 hours. The precipitated solid was collected by filtration, washed with ether, then industrial methylated spirit, then ether again to give 2-(4-chlorophenyl)-4-methyl[1]benzopyrano[4,3-c]pyrazol-3(2H)-one, m.p. 203-206° C.

Example 96

A mixture of 4-methyl-2-(4-methylphenyl)-1,4-dihydro[1]benzopyrano[4,3-c]pyrazol-3(2H)-one (200 mg) (Example 105), p-chloroanil (0.15 g) and dimethyl sulphoxide (2 ml) was stirred at ambient temperature for 3 hours and then filtered. The product collected was slurried with dimethyl sulphoxide, cooled and filtered to

give 4-methyl-2-(4-methylphenyl)[1]benzopyrano[4,3-c]pyrazol-3(2H)-one, m.p. 157-160° C.

Example 97

A stirred suspension of 4-methyl-2-(4-trifluoromethylphenyl)[1]benzopyrano[4,3-c]pyrazol-3(2H)-one (1.10 g) (Example 47) in ethanol (22 ml) was treated with sodium borohydride (0.24 g) at 0-5° C. The mixture was stirred for 20 minutes while allowed to warm up to ambient temperature. The ethanol was decanted off and added to a mixture of water (180 ml) and petrol (b.p. 60-80° C, 20 ml). The pH was adjusted to 4-5 with glacial acetic acid and the solid collected by filtration. This solid was purified by flash chromatography on silica (to remove starting material) using ethyl acetate/glacial acetic acid (9:1) to elute the column. This gave 4-methyl-2-(4-trifluoromethylphenyl)-1,4-dihydro[1]benzopyrano[4,3-c]pyrazol-3(2H)-one, m.p. 143-145° C.

Example 98

A stirred suspension of 2-(3-chlorophenyl)-4-methyl[1]benzopyrano[4,3-c]pyrazol-3(2H)-one (1.3 g) (Example 38) in ethanol (31 ml) at 0-5° C was treated with sodium borohydride (0.33 g). The reaction mixture was stirred at this temperature for 22 minutes and then poured onto a stirred mixture of water (9 volumes) and petrol (b.p. 60-80° C, 1 volume). After decantation to remove insoluble starting material, the pH of the mixture was adjusted to 4-5 with glacial acetic acid and the mixture was filtered to give 2-(3-chlorophenyl)-4-methyl-1,4-dihydro[1]benzopyrano[4,3-c]pyrazol-3(2H)-one, m.p. 140-141° C.

Example 99

A stirred mixture of 2-(3,4-dichlorophenyl)-4-methyl[1]benzopyrano[4,3-c]pyrazol-3(2H)-one (1.31 g) (Example 36) in ethanol (28.5 ml) at 0-5° C was treated portionwise with sodium borohydride (0.29 g) and the resulting mixture stirred at this temperature for 22 minutes. The solution was decanted from a small amount of solid and added to a cooled, stirred mixture of water and petrol (b.p. 60-80° C, 9:1). The pH of the mixture was adjusted to 4-5 with glacial acetic acid after which it was filtered. The collected solid product was washed, dried and recrystallised from industrial methylated spirit to give 2-(3,4-dichlorophenyl)-4-methyl-1,4-dihydro[1]benzopyrano[4,3-c]pyrazol-3(2H)-one, m.p. 147-150° C.

Example 100

A stirred suspension of 2-(4-chlorophenyl)-4-methyl[1]benzopyrano[4,3-c]pyrazol-3(2H)-one (2.04 g) (Example 91b) in ethanol (49 ml) at 0-5° C was treated portionwise with sodium borohydride (0.76 g). The reaction mixture was stirred at this temperature for 15 minutes and then poured into ice-cooled water. The pH of the mixture was adjusted to 4-5 with glacial acetic acid and the mixture was filtered. The collected solid product was washed with water, dried and recrystallised from methanol to give 2-(4-chlorophenyl)-4-methyl-1,4-dihydro[1]benzopyrano[4,3-c]pyrazol-3(2H)-one, m.p. 143-147° C.

Example 101

A stirred mixture of 2-(4-fluorophenyl)-4-methyl[1]benzopyrano[4,3-c]pyrazol-3(2H)-one (2.63 g) (Example 37) in ethanol (67 ml) at 0-5° C was treated portionwise with sodium borohydride (0.67 g) during 5 minutes. The reaction mixture was stirred at this temperature for 0.5 hours and then added to a cooled, stirred mixture of water and petrol (b.p. 62-68° C, 9:1). The pH of the mixture was adjusted to 4-5 with glacial acetic acid and the resulting mixture filtered. The collected solid product was washed and dried to give 2-(4-fluorophenyl)-4-methyl-1,4-dihydro[1]benzopyrano[4,3-c]pyrazol-3(2H)-one as its monohydrate, m.p. 114-117° C.

Example 102

a) A mixture of ethyl 2-(4-chlorophenyl)-3-oxo-2,3-dihydro[1]benzopyrano[4,3-c]pyrazole-4-acetate (100 mg) (Example 41) and 4-methoxybenzyl alcohol (0.32 ml) was stirred and heated at 150 °C for 50 minutes. The mixture was cooled to ambient temperature, diluted with ether and filtered to give 4-methoxybenzyl 2-(4-chlorophenyl)-3-oxo-2,3-dihydro[1]benzopyrano[4,3-c]pyrazole-4-acetate, m.p. 161-163 °C.

b) A mixture of 4-methoxybenzyl-2-(4-chlorophenyl)-3-oxo-2,3-dihydro[1]benzopyrano[4,3-c]pyrazole-4-acetate (1.38 g) and dichloromethane (7 ml) was stirred at 0 °C and methoxybenzene (0.28 ml) and trifluoroacetic acid (3.45 ml) were added. The mixture was stirred for 110 minutes at 0 °C then filtered. The filtrate was diluted with ice-cold water and the solid formed was collected by filtration, washed with water and dried. This solid was stirred with ether (10 ml) for 5 minutes then filtered to give 2-(4-chlorophenyl)-3-oxo-2,3-dihydro[1]benzopyrano[4,3-c]pyrazole-4-acetic acid, m.p. 195-198 °C.

Example 103

A mixture of 2-(4-chlorophenyl)-4-methyl[1]benzopyrano[4,3-c]pyrazol-3(2H)-one (2.0 g) (Example 91b) in propan-2-ol (36 ml) was treated with 2M sodium hydroxide (3.2 ml) with stirring at ambient temperature. After stirring for 10 minutes the mixture was poured into water and acidified with 5M hydrochloric acid. This mixture was extracted with dichloromethane and on washing the combined extracts with water, a solid was formed which was collected by filtration and dried to give 1-(4-chlorophenyl)-4-{1-[2-(4-chlorophenyl)-3-hydroxy-2,4-dihydro[1]benzopyrano[4,3-c]pyrazol-4-ylidenemethyl]-ethylidene}-3-(2-hydroxyphenyl)-2-pyrazolin-5-one, m.p. 220-222 °C.

Example 104

In a similar manner to Example 103 a mixture of 2-(3,4-dichlorophenyl)-4-methyl[1]benzopyrano[4,3-c]-pyrazol-3(2H)-one (Example 36) (1.43 g) in propan-2-ol (23.5 ml) was treated with 2M sodium hydroxide (2.1 ml) with stirring for 15 minutes. After acidification the solid formed was collected by filtration and dried. The solid was stirred with dichloromethane (10 ml) for 10 minutes, then collected by filtration to give 1-(3,4-dichlorophenyl)-4-{1-[2-(3,4-dichlorophenyl)-3-hydroxy-2,4-dihydro[1]benzopyrano[4,3-c]pyrazol-4-ylidenemethyl]ethylidene}-3-(2-hydroxyphenyl)-2-pyrazolin-5-one, m.p. 213-215 °C.

Example 105

In a similar manner to Example 101, 4-methyl-2-(4-methylphenyl) [1]benzopyrano[4,3-c]pyrazol-3(2H)-one (Example 43) (2.12 g) in ethanol (50 ml) was reacted with sodium borohydride (0.56 g) to give, after recrystallisation from industrial methylated spirit, 4-methyl-2-(4-methylphenyl)-1,4-dihydro[1]benzopyrano-[4,3-c]pyrazol-3(2H)-one, m.p. 118-121 °C.

Example 106

In the preparation of tablets comprising 100 parts by weight of active compound, 250 parts by weight lactose, 22 parts by weight maize starch, 10 parts by weight polyvinylpyrrolidone and 3 parts by weight magnesium stearate, the active compound, the lactose and some of the starch are de-aggregated, blended and the resulting mixture is granulated with a solution of polyvinylpyrrolidone in ethanol. The dry granulate is blended with the magnesium stearate and the rest of the starch. The mixture is then compressed in a tableting machine to give tablets containing 100mg active compound.

**Claims**

1. A compound of formula I

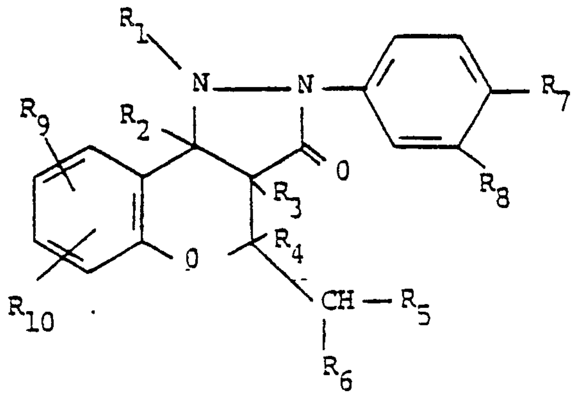

I

wherein $R_1$ represents hydrogen or together with $R_2$ represents a bond; $R_2$ together with either one of $R_1$ and $R_3$ represents a bond; $R_3$ together with either one of $R_2$ and $R_4$ represents a bond; $R_4$ represents hydrogen or together with $R_3$ represents a bond; $R_5$ represents hydrogen or methyl; $R_6$ represents hydrogen, halo, a $C_{2-5}$ alkanoyl group, a $C_{2-5}$ alkoxycarbonyl group, a $C_{1-6}$ alkylthio group, a $C_{1-6}$ alkylsulphinyl group, a $C_{1-5}$ alkylsulphonyl group, carbamoyl, carboxy or $R_5$ and $R_6$ together with the carbon atom to which they are attached represent a cyclopropyl group;$R_7$ represents hydrogen, halo, trifluoromethyl, methoxy, a $C_{1-5}$ alkyl group, a $C_{1-6}$ alkylthio group or a $C_{1-6}$ alkylsulphinyl group; $R_8$ represents hydrogen, halo or trifluoromethyl; $R_9$ and $R_{10}$, which may be the same or different, each represent halo; or $R_9$ represents hydrogen and $R_{10}$ represents hydrogen, halo, trifluoromethyl, hydroxy, nitro, a $C_{2-6}$ alkanoyloxy group, a $C_{1-6}$ alkyl group or a $C_{1-6}$ alkoxy group provided that when $R_1$ and $R_2$ represent a bond, $R_3$ and $R_4$ represent a bond, and $R_6$, $R_7$, $R_8$, $R_9$ and $R_{10}$ each represent hydrogen, then $R_5$ is not hydrogen.

2. A compound according to claim 1 wherein $R_5$ represents hydrogen, $R_6$ represents hydrogen, halo, a $C_{2-6}$ alkanoyl group, a $C_{2-5}$ alkoxycarbonyl group, a $C_{1-6}$ alkylthio group, a $C_{1-6}$ alkylsulphinyl group, a $C_{1-5}$ alkylsulphonyl group, carbamoyl, carboxy or $R_5$ and $R_6$ together with the carbon atom to which they are attached represent a cyclopropyl group; $R_7$ represents hydrogen, fluoro, chloro, trifluoromethyl, a $C_{1-6}$ alkylthio group or a $C_{1-5}$ alkylsulphinyl group; $R_8$ represents hydrogen or chloro; and $R_9$ and $R_{10}$ each represent fluoro, or $R_9$ represents hydrogen and $R_{10}$ is a substituent in the 8- or 9- position of the benz ring and represents hydrogen, fluoro, trifluoromethyl, hydroxy, nitro, a $C_{2-6}$ alkanoyloxy group or a $C_{1-6}$ alkyl group, or $R_{10}$ represents a $C_{1-5}$ alkoxy group in position 9- of the benz ring.

3. A compound according to either one of claims 1 and 2 wherein $R_6$ represents hydrogen, a $C_{2-6}$ alkoxycarbonyl group, a $C_{1-5}$ alkylthio group, a $C_{1-6}$ alkylsulphinyl group, a $C_{1-6}$ alkylsulphonyl group, carbamoyl, carboxy or $R_5$ and $R_6$ together with the carbon atom to which they are attached represent a cyclopropyl group; $R_7$ represents hydrogen, fluoro, chloro, trifluoromethyl, a $C_{1-6}$ alkylthio group or a $C_{1-6}$ alkylsulphinyl group; $R_8$ represents hydrogen or chloro; and $R_9$ and $R_{10}$ each represent fluoro, or $R_9$ represents hydrogen and $R_{10}$ is a substituent in the 8- or 9- position of the benz ring and represents hydrogen, fluoro, trifluoromethyl, hydroxy, nitro, a $C_{1-6}$ alkyl group, or $R_{10}$ represents a $C_{1-6}$ alkoxy group in position 9- of the benz ring.

4. A compound according to either one of claims 1 and 2 wherein $R_5$ represents hydrogen, $R_6$ represents hydrogen, halo, a $C_{2-4}$ alkanoyl group, a $C_{2-6}$ alkoxycarbonyl group, a $C_{1-4}$ alkylthio group, a $C_{1-4}$ alkylsulphinyl group, a $C_{1-4}$ alkylsulphonyl group, carbamoyl, carboxy or $R_5$ and $R_6$ together with the carbon atom to which they are attached represent a cyclopropyl group.

5. A compound according to claim 4 wherein $R_6$ represents hydrogen, chloro, bromo, acetyl, a $C_{2-4}$ alkoxycarbonyl group, methylthio, ethylthio, methylsulphinyl, methylsulphonyl, carbamoyl, carboxy or $R_6$ together with $R_5$ and the carbon atom to which they are attached represent a cyclopropyl group.

6. A compound according to any one of the preceding claims wherein $R_6$ represents hydrogen, methoxycarbonyl, ethoxycarbonyl, propoxycarbonyl, or methylthio.

7. A compound according to any one of the preceding claims wherein $R_6$ represents hydrogen or propoxycarbonyl.

8. A compound according to any one of the preceding claims wherein $R_6$ represents hydrogen.

9. A compound according to any one of the preceding claims wherein $R_7$ represents hydrogen, fluoro, chloro, trifluoromethyl, a $C_{1-4}$ alkylthio group or a $C_{1-4}$ alkylsulphinyl group.

10. A compound according to any one of the preceding claims wherein $R_7$ represents hydrogen, fluoro, chloro, trifluoromethyl, methylthio or methylsulphinyl.

11. A compound according to any one of the preceeding claims wherein $R_7$ represents fluoro, chloro, trifluoromethyl or methylthio.

12. A compound according to any one of the preceding claims wherein $R_7$ represents fluoro or chloro.

13. A compound according to any one of the preceding claims wherein $R_8$ represents hydrogen or chloro.

14. A compound according to any one of claims 1, 2 or 4 to 13 wherein $R_9$ represents hydrogen and $R_{10}$ represents hydrogen, fluoro, hydroxy, nitro, a $C_{2-6}$ alkanoyloxy group or a $C_{1-4}$ alkoxy group.

15. A compound according to claim 14 wherein $R_{10}$ represents hydrogen, fluoro, hydroxy, a $C_{2-4}$ alkanoyloxy group or a $C_{1-4}$ alkoxy group.

16. A compound according to claim 15 wherein $R_{10}$ represents hydrogen or fluoro or a $C_{2-4}$ alkanoyloxy group in position 8- of the benz ring or hydroxy or a $C_{1-4}$ alkoxy group in position 9- of the benz ring.

17. A compound according to any one of the preceding claims wherein $R_9$ represents hydrogen and $R_{10}$ represents hydrogen or 8-fluoro.

18. A compound according to claim 1 represented by formula II

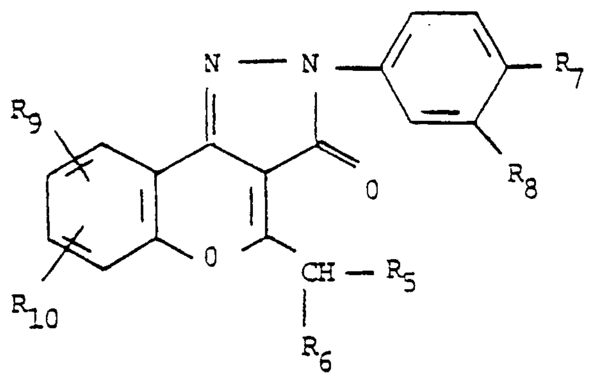

II

wherein $R_5$ represents hydrogen, $R_6$ represents hydrogen, methoxycarbonyl, ethoxycarbonyl, propoxycarbonyl, or methylthio, $R_7$ represents fluoro, chloro, bromo, trifluoromethyl or methylthio, $R_8$ represents hydrogen or chloro, $R_9$ represents hydrogen, $R_{10}$ represents hydrogen, 8-fluoro, 8-propionyloxy, 9-hydroxy or 9-ethoxy.

19. A compound according to claim 1 represented by formula III

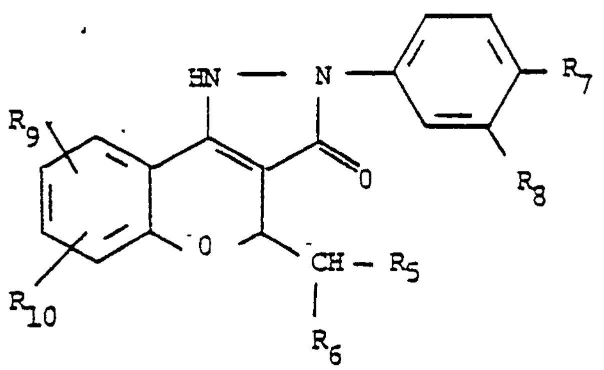

III

wherein $R_5$ and $R_6$ represent hydrogen, $R_7$ represents hydrogen or chloro, $R_8$ represents hydrogen or chloro and $R_9$ and $R_{10}$ each represent hydrogen.

20. A compound selected from :-
2-(4-chlorophenyl)-4-methyl[1]benzopyrano[4,3-c]pyrazol-3(2H)-one,
ethyl 2-(4-chlorophenyl)-3-oxo-2,3-dihydro[1]benzopyrano[4,3-c]pyrazol-4-acetate,
propyl 2-(4-chlorophenyl)-3-oxo-2,3-dihydro[1]benzopyrano[4,3-c]pyrazol-4-acetate,
isopropyl 2-(4-chlorophenyl)-3-oxo-2,3-dihydro[1]benzopyrano[4,3-c]pyrazol-4-acetate.

21. A pharmaceutical composition comprising a compound of formula I

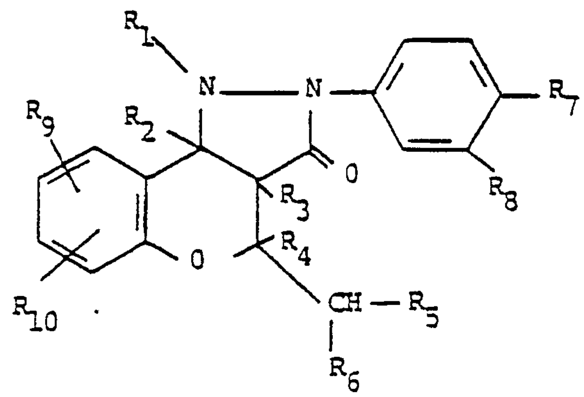

I

wherein $R_1$ represents hydrogen or together with $R_2$ represents a bond; $R_2$ together with either one of $R_1$ and $R_3$ represents a bond; $R_4$ together with either one of $R_2$ and $R_4$ represents a bond; $R_4$ represents hydrogen or together with $R_3$ represents a bond; $R_5$ represents hydrogen or methyl; $R_6$ represents hydrogen, halo, a $C_{2-5}$ alkanoyl group, a $C_{2-5}$ alkoxycarbonyl group, a $C_{1-6}$ alkylthio group, a $C_{1-6}$ alkylsulphinyl group, a $C_{1-6}$ alkylsulphonyl group, carbamoyl, carboxy, or $R_5$ and $R_6$ together with the carbon atom to which they are attached represent a cyclopropyl group;$R_7$ represents hydrogen, halo, trifluoromethyl, methoxy, a $C_{1-6}$ alkyl group, a $C_{1-6}$ alkylthio group or a $C_{1-6}$ alkylsulphinyl group; $R_8$ represents hydrogen, halo or trifluoromethyl $R_9$ and $R_{10}$, which may be the same or different, each represent halo; or $R_9$ represents hydrogen and $R_{10}$ represents hydrogen, halo, trifluoromethyl, hydroxy, nitro, a $C_{2-6}$ alkanoyloxy group, a $C_{1-6}$ alkyl group or a $C_{1-6}$ alkoxy group, together with a pharmaceutically acceptable carrier.

22. A pharmaceutical composition according to claim 21 comprising a compound of formula I as claimed in any one of claims 2 to 20.

23. A pharmaceutical composition according to either one of claims 21 and 22 in unit dosage form.

24. A method of treating diseases with an immunological association in a mammal in need of such treatment comprising the administration of a therapeutically effective amount of a compound of formula I as defined in claim 21.

25. A method according to claim 24 comprising the administration of a therapeutically effective amount of a compound of formula I as claimed in any one of claims 2 to 20.

26. A compound of formula I as defined in claim 21 for use as an immunomodulatory agent.

27. A compound of formula I as claimed in any one of claims 2 to 20 for the use according to claim 26.

28. A compound of formula I as claimed in claim 21 in the manufacture of a medicament for use in the treatment of diseases with an immunological association.

29. A compound of formula I as claimed in any one of claims 2 to 20 for the use according to claim 28.

30. A process to prepare a compound of formula I wherein $R_1$ and $R_2$ form a bond, $R_3$ and $R_4$ form a bond and $R_5$, $R_6$, $R_7$, $R_8$, $R_9$ and $R_{10}$ are as defined in claim 21, comprising oxidising a compound of formula I wherein $R_1$ represents hydrogen, $R_2$ and $R_3$ represent a bond and $R_4$ represents hydrogen and $R_5$, $R_6$, $R_7$, $R_8$, $R_9$ and $R_{10}$ are as herein defined.

31. A process to prepare a compound of formula I wherein $R_1$ and $R_2$ form a bond, $R_3$ and $R_4$ form a bond and $R_5$, $R_6$, $R_7$, $R_8$, $R_9$ and $R_{10}$ are as defined in claim 21, comprising reacting a compound of formula IV

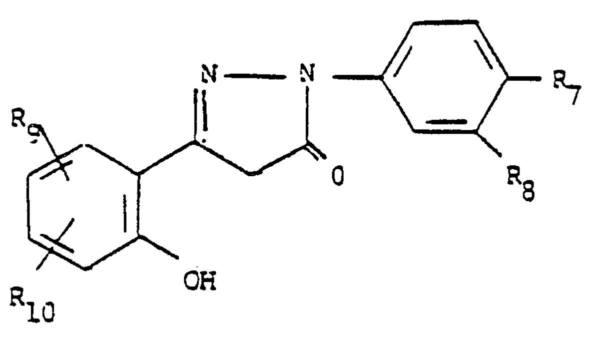

IV

or a tautomer thereof, with an orthoester of formula V(a)

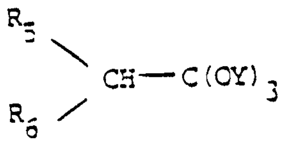

V(a)

or a thio ortho ester of formula V(b)

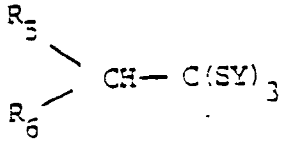

V(b)

wherein Y represents a $C_{1-4}$ alkyl group or a benzyl group.

32. A process to prepare a compound of formula I wherein $R_1$ and $R_2$ form a bond, $R_3$ and $R_4$ form a bond and $R_5$, $R_6$, $R_7$, $R_8$, $R_9$ and $R_{10}$ are as defined in claim 21, comprising reacting a compound of formula VI

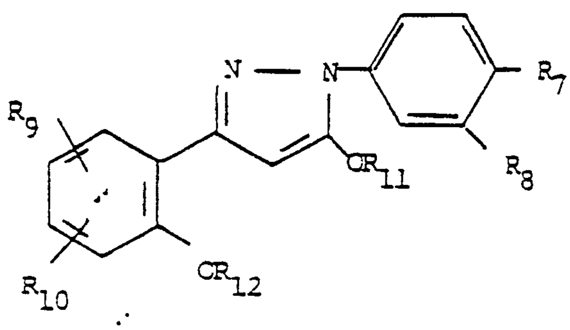

VI

wherein $R_{11}$ represents hydrogen, or a tautomer thereof, or in which $R_{11}$ represents the group $COR_{13}$, $R_{12}$ represents $COCHR_5R_6$, and $R_{13}$ represents hydrogen, a $C_{1-4}$ alkyl group or a benzyl group, with a base.

33. A process to prepare a compound of formula I wherein $R_1$ represents hydrogen, $R_2$ and $R_3$ represent a bond, $R_4$ represents hydrogen and $R_5$, $R_6$, $R_7$, $R_8$, $R_9$ and $R_{10}$ are as defined in claim 21, comprising reducing compounds of formula I wherein $R_1$ and $R_2$ represent a bond, $R_3$ and $R_4$ form a bond and $R_5$, $R_6$, $R_7$, $R_8$, $R_9$ and $R_{10}$ are as herein defined.

34. A process to prepare a compound of formula I wherein $R_1$ represents hydrogen, $R_2$ and $R_3$ represent a bond, $R_4$ represents hydrogen and $R_5$, $R_6$, $R_7$, $R_8$, $R_9$ and $R_{10}$ are as defined in claim 21, comprising reacting a compound of formula VII

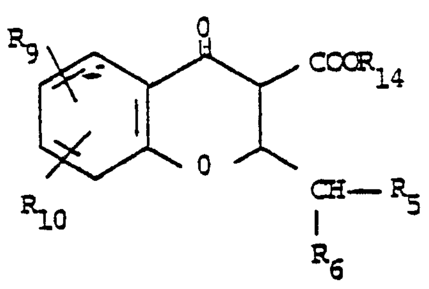

VII

in which $R_{14}$ represents a $C_{1-4}$ alkyl group, with a compound of formula VIII

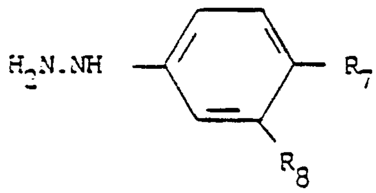

VIII

35. A compound of formula XVI

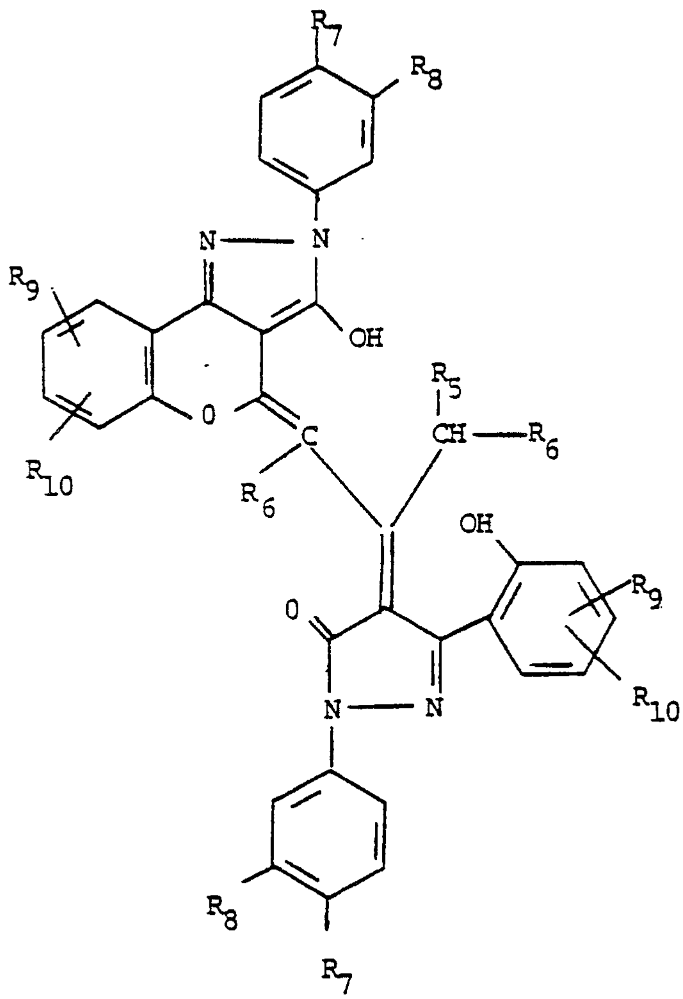

XVI

wherein $R_5$, $R_6$, $R_7$, $R_8$, $R_9$ and $R_{10}$ are as defined in claim 21.

Claims for the following Contracting States: GR, ES

1. A process to prepare a compound of formula I

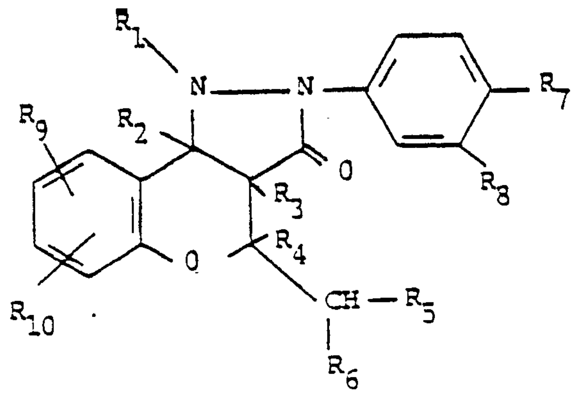

I

wherein $R_1$ and $R_2$ form a bond, $R_3$ and $R_4$ form a bond; $R_5$ represents hydrogen or methyl; $R_6$ represents hydrogen, halo, a $C_{2-6}$ alkanoyl group, a $C_{2-6}$ alkoxycarbonyl group, a $C_{1-6}$ alkylthio group, a $C_{1-6}$ alkylsulphinyl group, a $C_{1-6}$ alkylsulphonyl group, carbamoyl, carboxy or $R_5$ and $R_6$ together with the carbon atom to which they are attached represent a cyclopropyl group;$R_7$ represents hydrogen, halo, trifluoromethyl, methoxy, a $C_{1-6}$ alkyl group, a $C_{1-6}$ alkylthio group or a $C_{1-6}$ alkylsulphinyl group; $R_8$ represents hydrogen, halo or trifluoromethyl; $R_9$ and $R_{10}$, which may be the same or different, each represent halo; or $R_9$ represents hydrogen and $R_{10}$ represents hydrogen, halo, trifluoromethyl, hydroxy, nitro, a $C_{2-6}$ alkanoyloxy group, a $C_{1-6}$ alkyl group or a $C_{1-6}$ alkoxy group

a) comprising oxidising a compound of formula I wherein $R_1$ represents hydrogen, $R_2$ and $R_3$ represent a bond and $R_4$ represents hydrogen and $R_5$, $R_6$, $R_7$, $R_8$, $R_9$ and $R_{10}$ are as herein defined;

b) comprising reacting a compound of formula IV

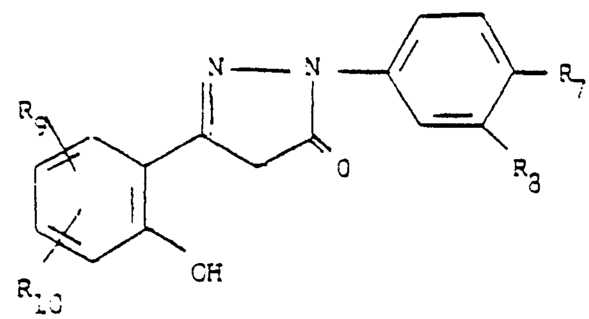

IV

or a tautomer thereof, with an orthoester of formula V(a)

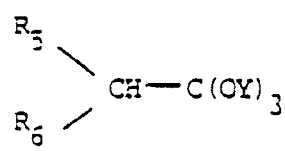

V(a)

or a thio ortho ester of formula V(b)

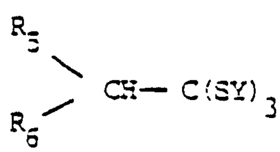

V(b)

wherein Y represents a $C_{1-4}$ alkyl group or a benzyl group; or

c) comprising reacting a compound of formula VI

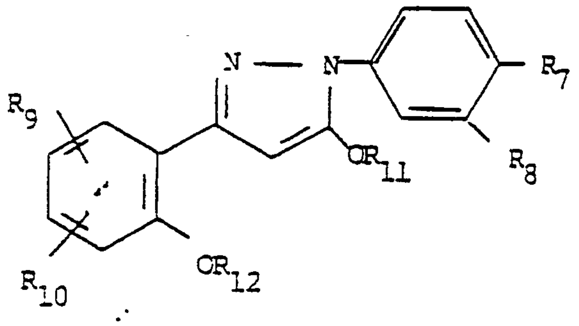

VI

wherein $R_{11}$ represents hydrogen, or a tautomer thereof, or in which $R_{11}$ represents the group $COR_{13}$, $R_{12}$ represents $COCHR_5R_6$, and $R_{13}$ represents hydrogen, a $C_{1-4}$ alkyl group or a benzyl group, with a base.

2. A process to prepare a compound of formula I wherein $R_1$ represents hydrogen, $R_2$ and $R_3$ represent a bond, $R_4$ represents hydrogen and $R_5$, $R_6$, $R_7$, $R_8$, $R_9$ and $R_{10}$ are as defined in claim 1,

a) comprising reducing compounds of formula I wherein $R_1$ and $R_2$ represent a bond, $R_3$ and $R_4$ form a bond and $R_5$, $R_6$, $R_7$, $R_8$, $R_9$ and $R_{10}$ are as herein defined; or

b) comprising reacting a compound of formula VII

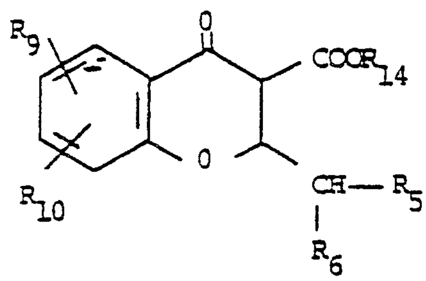

VII

in which $R_{14}$ represents a $C_{1-4}$ alkyl group, with a compound of formula VIII

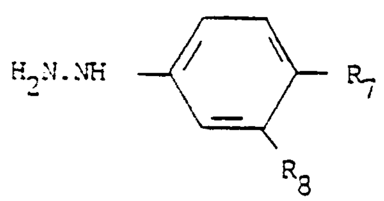

VIII

3. A process according to either one of claims 1 and 2 wherein $R_6$ represents hydrogen, a $C_{2-6}$ alkoxycarbonyl group, a $C_{1-6}$ alkylthio group, a $C_{1-6}$ alkylsulphinyl group, a $C_{1-6}$ alkylsulphonyl group, carbamoyl, carboxy or $R_5$ and $R_6$ together with the carbon atom to which they are attached represent a cyclopropyl group; $R_7$ represents hydrogen, fluoro, chloro, trifluoromethyl, a $C_{1-6}$ alkylthio group or a $C_{1-6}$ alkylsulphinyl group; $R_8$ represents hydrogen or chloro; and $R_9$ and $R_{10}$ each represent fluoro, or $R_9$ represents hydrogen and $R_{10}$ is a substituent in the 8- or 9- position of the benz ring and represents hydrogen, fluoro, trifluoromethyl, hydroxy, nitro, a $C_{1-6}$ alkyl group, or $R_{10}$ represents a $C_{1-6}$ alkoxy group in position 9- of the benz ring.

4. A process according to claim 1 to prepare compounds represented by formula II

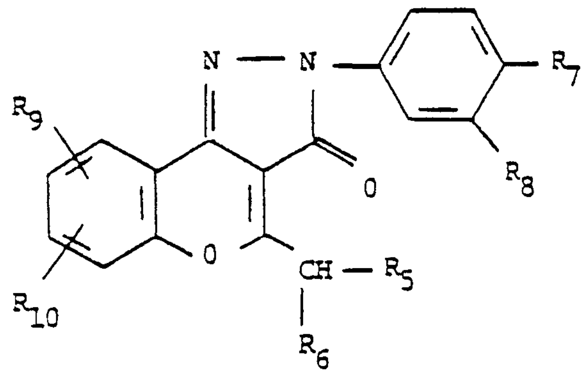

II

wherein $R_5$ represents hydrogen, $R_6$ represents hydrogen, methoxycarbonyl, ethoxycarbonyl, propoxycarbonyl, or methylthio, $R_7$ represents fluoro, chloro, bromo, trifluoromethyl or methylthio, $R_8$ represents hydrogen or chloro, $R_9$ represents hydrogen, $R_{10}$ represents hydrogen, 8-fluoro, 8-propionyloxy, 9-hydroxy or 9-ethoxy.

5. A process according to claim 2 to prepare compounds represented by formula III

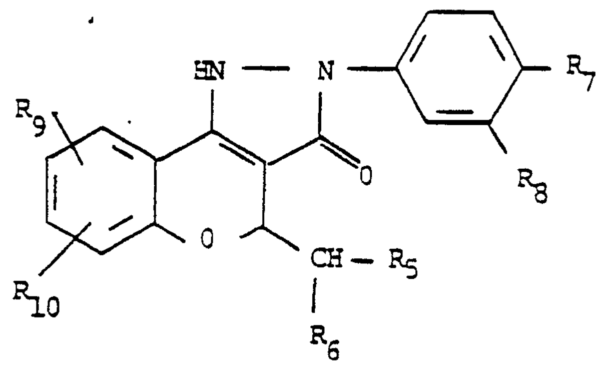

III

wherein $R_5$ and $R_6$ represent hydrogen, $R_7$ represents hydrogen or chloro, $R_8$ represents hydrogen or chloro and $R_9$ and $R_{10}$ each represent hydrogen.

European Patent Office

# PARTIAL EUROPEAN SEARCH REPORT

which under Rule 45 of the European Patent Convention shall be considered, for the purposes of subsequent proceedings, as the European search report

Application number
EP 89 30 7724

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | CHEMICAL ABSTRACTS, vol. 106, 1987, page 619, ref. 119619f, Columbus, Ohio, US; C.K. GHOSH et al.: "Benzopyrans. Part XX. 4-Oxo-4H(1)benzopyran-3-carbonitrile/carboxylic acid: change of their reaction courses by a methyl substituent at the 2-position" & INDIAN J. CHEM., SECT. B 1985, 24B(12)<br>* Formula III *<br>-- | 1-10, 13,15 | C 07 D 491/052<br>A 61 K 31/415//<br>(C 07 D 491/052,<br>C 07 D 335:00,<br>C 07 D 231:00) |
| D,A | US-A-4 268 516 (PFIZER)<br>* Claims 1,11 *<br>--------- | 1,21 | |
| | | | TECHNICAL FIELDS SEARCHED (Int. Cl.4) |
| | | | C 07 D 491/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-23,26-35

Claims searched incompletely:

Claims not searched: 24,25

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 30-10-1989 | ALFARO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03 82